# EUROPEAN PATENT SPECIFICATION

(11) **EP 1 826 217 B1**
(45) Date of publication and mention of the grant of the patent: **21.07.2010**
(21) Application number: 07011493.9
(22) Date of filing: 24.09.2003
(51) Int. Cl.: C07K 14/705, A61K 39/00, A61K 38/17, G01N 33/68

(54) **Method for identification of antigenic peptides**
Verfahren zur Identifizierung antigener Peptide
Procédé d'identification des peptides antigéniques

(30) Priority: 02.10.2002 EP 02022223
(43) Date of publication of application: 29.08.2007
(62) Divisional of application: 03021521.4
(73) Proprietor: F.HOFFMANN-LA ROCHE AG, 4070 Basel (CH)
(72) Inventor: Kropshofer, Harald, 79539 Lörrach (DE); Vogt, Anne, 79539 Lörrach (DE)

(56) References cited:
- WO-A-02/40647
- WO-A1-00/05250
- WO-A2-00/05249
- KROPSHOFER H ET AL: "SELF-RELEASE OF CLIP IN PEPTIDE LOADING OF HLA-DR MOLECULES" SCIENCE, AMERICAN ASSOCIATION FOR THE ADVANCEMENT OF SCIENCE,, US, vol. 270, no. 5240, November 1995 (1995-11), pages 1357-1359, XP008026441 ISSN: 0036-8075
- KROPSHOFER H ET AL: "Self and Foreign Peptides Interact with Intact and Disassembled MHC Class II Antigen HLA-DR via Tryptophan Pockets" BIOCHEMISTRY, vol. 30, 1991, pages 9177-9187, XP002442893
- KALBACHER H & KROPSHOFER H: "Non-radioactive detection of MHC class II-peptide antigen complexes in the sub-picomole range by high-performance size-exclusion chromatography with fluorescence detection" JOURNAL OF CHROMATOGRAPHY, vol. 548, 1991, pages 343-350, XP002406304
- PASSMORE D ET AL: "PREPARATIVE-SCALE PURIFICATION AND CHARACTERIZATION OF MHC CLASS II MONOMERS" JOURNAL OF IMMUNOLOGICAL METHODS, ELSEVIER SCIENCE PUBLISHERS B.V.,AMSTERDAM, NL, vol. 155, no. 2, 5 November 1992 (1992-11-05), pages 193-200, XP002026330 ISSN: 0022-1759
- BURROWS G G ET AL: "MULTIPLE CLASS I MOTIFS REVEALED BY SEQUENCING NATURALLY PROCESSED PEPTIDES ELUTED FROM RAT T CELL MHC MOLECULES" JOURNAL OF NEUROSCIENCE RESEARCH, WILEY-LISS, US, vol. 49, no. 1, 1997, pages 107-116, XP008026443 ISSN: 0360-4012
- DONGRE A R ET AL: "In vivo MHC class II presentation of cytosolic proteins revealed by rapid automated tandem mass spectrometry and functional analyses" EUROPEAN JOURNAL OF IMMUNOLOGY, WEINHEIM, DE, vol. 31, no. 5, May 2001 (2001-05), pages 1485-1494, XP002304910 ISSN: 0014-2980
- KROPSHOFER H ET AL: "Tetraspan microdomains distinct from lipid rafts enrich select peptide-MHC class II complexes" NATURE IMMUNOLOGY, vol. 3, no. 1, January 2002 (2002-01), pages 61-68, XP002442800 ISSN: 1529-2908
- RÖHN TA ET AL: "A Novel Strategy for the Discovery of MHC Class II -Restricted Tumor Antigens: Identification of a Melanotransferrin Helper T-Cell Epitope" CANCER RESEARCH, vol. 65, no. 21, 1 November 2005 (2005-11-01), pages 10068-10078, XP002442801 ISSN: 0008-5472

### SEQUENCE LISTING

<110> F. Hoffmann-La Roche Ltd.
<120> Method for the identification of antigenic peptides associated to diseases
<130> 21388
<160> 90
<170> PatentIn version 3.2
<210> 1
   <211> 16
   <212> PRT
   <213> Homo sapiens
<400> 1
   Ser Lys Glu Gln Leu Thr Pro Leu Ile Lys Lys Ala Gly Thr Glu Leu
   1               5                   10                  15
<210> 2
   <211> 15
   <212> PRT
   <213> Homo sapiens
<400> 2
   Ser Lys Glu Gln Leu Thr Pro Leu Ile Lys Lys Ala Gly Thr Glu
   1               5                   10                  15
<210> 3
   <211> 14
   <212> PRT
   <213> Homo sapiens
<400> 3
   Lys Glu Gln Leu Thr Pro Leu Ile Lys Lys Ala Gly Thr Glu
   1 5 10
<210> 4
   <211> 16
   <212> PRT
   <213> Homo sapiens
<400> 4
   Ser Val Leu Leu Phe Ile Glu His Ser Val Glu Val Ala His Gly Lys
   1 5 10 15
<210> 5
   <211> 17
   <212> PRT
   <213> Homo sapiens
<400> 5
   Val Asp Gly Ile Leu Ser Asn Cys Gly Ile Glu Lys Glu Ser Asp Leu
   1 5 10 15
   Cys
<210> 6
   <211> 17
   <212> PRT
   <213> Homo sapiens
<400> 6
   Gly Thr Gln Gly Lys Ile Val Asp Leu Val Lys Glu Leu Asp Arg Asp
   1 5 10 15
   Thr
<210> 7
   <211> 16
   <212> PRT
   <213> Homo sapiens
<400> 7
   Thr Gln Gly Lys Ile Val Asp Leu Val Lys Glu Leu Asp Arg Asp Thr
   1 5 10 15
<210> 8
   <211> 14
   <212> PRT
   <213> Homo sapiens
<400> 8
   Thr Gln Gly Lys Ile Val Asp Leu Val Lys Glu Leu Asp Arg
   1 5 10
<210> 9
   <211> 14
   <212> PRT
   <213> Homo sapiens
<400> 9
   Gly Lys Asn Ile Lys Ile Ile Ser Lys Ile Glu Asn His Glu
   1 5 10
<210> 10
   <211> 14
   <212> PRT
   <213> Homo sapiens
<400> 10 <210> 11
   <211> 22
   <212> PRT
   <213> Homo sapiens
<400> 11
<210> 12
   <211> 18
   <212> PRT
   <213> Homo sapiens
<400> 12
   Ser Thr Pro Thr Leu Val Glu Val Ser Arg Asn Leu Gly Lys Val Gly
   1 5 10 15
   Ser Lys
<210> 13
   <211> 17
   <212> PRT
   <213> Homo sapiens
<400> 13
   Thr Pro Thr Leu Val Glu Val Ser Arg Asn Leu Gly Lys Val Gly Ser
   1 5 10 15
   Lys
<210> 14
   <211> 17
   <212> PRT
   <213> Homo sapiens
<400> 14
   Val Ser Thr Pro Thr Leu Val Glu Val Ser Arg Asn Leu Gly Lys Val
   1 5 10 15
   Gly
<210> 15
   <211> 17
   <212> PRT
   <213> Homo sapiens
<400> 15
   Ser Thr Pro Thr Leu Val Glu Val Ser Arg Asn Leu Gly Lys Val Gly
   1 5 10 15
   Ser
<210> 16
   <211> 16
   <212> PRT
   <213> Homo sapiens
<400> 16
   Ser Thr Pro Thr Leu Val Glu Val Ser Arg Asn Leu Gly Lys Val Gly
   1 5 10 15
<210> 17
   <211> 15
   <212> PRT
   <213> Homo sapiens
<400> 17
   Thr Pro Thr Leu Val Glu Val Ser Arg Asn Leu Gly Lys Val Gly
   1 5 10 15
<210> 18
   <211> 14
   <212> PRT
   <213> Homo sapiens
<400> 18
   Thr Pro Thr Leu Val Glu Val Ser Arg Asn Leu Gly Lys Val
   1 5 10
<210> 19
   <211> 16
   <212> PRT
   <213> Homo sapiens
<400> 19
   Gly Lys Asp Tyr Ile Ala Leu Asn Glu Asp Leu Arg Ser Trp Thr Ala
   1 5 10 15
<210> 20
   <211> 16
   <212> PRT
   <213> Homo sapiens
<400> 20
   Gly Tyr Pro Thr Ile Lys Ile Leu Lys Lys Gly Gln Ala Val Asp Tyr
   1 5 10 15
<210> 21
   <211> 15
   <212> PRT
   <213> Homo sapiens
<400> 21
   Tyr Pro Thr Ile Lys Ile Leu Lys Lys Gly Gln Ala Val Asp Tyr
   1 5 10 15
<210> 22
   <211> 15
   <212> PRT
   <213> Homo sapiens
<400> 22
   Gly Tyr Pro Thr Ile Lys Ile Leu Lys Lys Gly Gln Ala Val Asp
   1 5 10 15
<210> 23
   <211> 14
   <212> PRT
   <213> Homo sapiens
<400> 23
   Tyr Pro Thr Ile Lys Ile Leu Lys Lys Gly Gln Ala Val Asp
   1 5 10
<210> 24
   <211> 16
   <212> PRT
   <213> Homo sapiens
<400> 24
   Ser Tyr Glu Leu Pro Asp Gly Gln Val Ile Thr Ile Gly Asn Glu Arg
   1 5 10 15
<210> 25
   <211> 14
   <212> PRT
   <213> Homo sapiens
<400> 25
   Ser Val Ile Leu Lys Ile Leu Pro Ser Tyr Gln Glu Pro His
   1 5 10
<210> 26
   <211> 15
   <212> PRT
   <213> Homo sapiens
<400> 26
   Ala Lys Ile His Ile Asp Ile Val Leu Val Gly Gly Ser Thr Arg
   1 5 10 15
<210> 27
   <211> 14
   <212> PRT
   <213> Homo sapiens
<400> 27
   Asn Ala Leu Leu Val Arg Thr Lys Lys Val Pro Gln Val Ser
   1 5 10
<210> 28
   <211> 15
   <212> PRT
   <213> Homo sapiens
<400> 28
   Arg Asn Gly Tyr Arg Ala Leu Met Asp Lys Ser Leu His Val Gly
   1 5 10 15
<210> 29
   <211> 15
   <212> PRT
   <213> Homo sapiens
<400> 29
   Met Asn Phe Ser Trp Ala Met Asp Leu Asp Phe Lys Gly Ala Asn
   1 5 10 15
<210> 30
   <211> 13
   <212> PRT
   <213> Homo sapiens
<400> 30
   Ser Tyr Leu Gln Asp Ser Asp Pro Asp Ser Phe Gln Asp
   1 5 10
<210> 31
   <211> 16
   <212> PRT
   <213> Homo sapiens
<400> 31
   Thr Leu Gln Ser Phe Arg Gln Asp Val Asp Asn Ala Ser Leu Ala Arg
   1 5 10 15
<210> 32
   <211> 17
   <212> PRT
   <213> Homo sapiens
<400> 32
   Phe Pro Glu Pro Ile Lys Leu Asp Asn Lys Asn Asp Arg Ala Lys Ala
   1 5 10 15
   Ser
<210> 33
   <211> 16
   <212> PRT
   <213> Homo sapiens
<400> 33
   His Thr Gly Ala Leu Tyr Arg Ile Gly Asp Leu Gln Ala Phe Gln Gly
   1 5 10 15
<210> 34
   <211> 14 <212> PRT
   <213> Homo sapiens
<400> 34
   Thr Gly Ala Leu Tyr Arg Ile Gly Asp Leu Gln Ala Phe Gln
   1 5 10
<210> 35
   <211> 16
   <212> PRT
   <213> Homo sapiens
<400> 35
   Ala Lys Asn Gln Val Ala Met Asn Pro Thr Asn Thr Val Phe Asp Ala
   1               5                   10                  15
<210> 36
   <211> 17
   <212> PRT
   <213> Homo sapiens
<400> 36
   Asn Val Leu Arg Ile Ile Asn Glu Pro Thr Ala Ala Ala Ile Ala Tyr
   1               5                   10                  15
   Gly
<210> 37
   <211> 18
   <212> PRT
   <213> Homo sapiens
<400> 37
   Leu Asn Val Leu Arg Ile Ile Asn Glu Pro Thr Ala Ala Ala Ile Ala
   1               5                   10                  15
   Tyr Gly
<210> 38
   <211> 14
   <212> PRT
   <213> Homo sapiens
<400> 38
   Ile Asp Lys Val Ile Ser Thr Ile Thr Asn Asn Ile Gln Gln
   1               5                   10
<210> 39
   <211> 14
   <212> PRT
   <213> Homo sapiens
<400> 39
   Asp Asp Val Ile Leu Asn Glu Pro Ser Ala Asp Ala Pro Ala
   1 5 10
<210> 40
   <211> 15
   <212> PRT
   <213> Homo sapiens
<400> 40
   Asn Ser Asn Gln Ile Lys Ile Leu Gly Asn Gln Gly Ser Phe Leu
   1 5 10 15
<210> 41
   <211> 16
   <212> PRT
   <213> Homo sapiens
<400> 41
   Asn Lys Glu Gly Leu Glu Leu Leu Lys Thr Ala Ile Gly Lys Ala Gly
   1 5 10 15
<210> 42
   <211> 15
   <212> PRT
   <213> Homo sapiens
<400> 42
   Lys Val Val Val Tyr Leu Gln Lys Leu Asp Thr Ala Tyr Asp Asp
   1 5 10 15
<210> 43
   <211> 16
   <212> PRT
   <213> Homo sapiens
<400> 43
   Lys Lys Val Val Val Tyr Leu Gln Lys Leu Asp Thr Ala Tyr Asp Asp
   1 5 10 15
<210> 44
   <211> 16
   <212> PRT
   <213> Homo sapiens
<400> 44
   Lys Val Val Val Tyr Leu Gln Lys Leu Asp Thr Ala Tyr Asp Asp Leu
   1 5 10 15
<210> 45
   <211> 15
   <212> PRT
   <213> Homo sapiens
<400> 45
   Tyr Pro Arg Ile Ser Val Asn Asn Val Leu Pro Val Phe Asp Asn
   1 5 10 15
<210> 46
   <211> 16
   <212> PRT
   <213> Homo sapiens
<400> 46
   Thr Thr Ala Phe Gln Tyr Ile Ile Asp Asn Lys Gly Ile Asp Ser Asp
   1 5 10 15
<210> 47
   <211> 17
   <212> PRT
   <213> Homo sapiens
<400> 47
   Thr Thr Ala Phe Gln Tyr Ile Ile Asp Asn Lys Gly Ile Asp Ser Asp
   1 5 10 15
   Ala
<210> 48
   <211> 16
   <212> PRT
   <213> Homo sapiens
<400> 48
   Leu Pro Gly Gln Leu Lys Pro Phe Glu Thr Leu Leu Ser Gln Asn Gln
   1 5 10 15
<210> 49
   <211> 17
   <212> PRT
   <213> Homo sapiens
<400> 49
   Leu Pro Gly Gln Leu Lys Pro Phe Glu Thr Leu Leu Ser Gln Asn Gln
   1               5                   10                  15
   Gly
<210> 50
   <211> 16
   <212> PRT
   <213> Homo sapiens
<400> 50
<210> 51
   <211> 17
   <212> PRT
   <213> Homo sapiens
<400> 51
   Val Asp Glu Val Thr Ile Val Asn Ile Leu Thr Asn Arg Ser Asn Ala
   1               5                   10                  15
   Gln
<210> 52
   <211> 17
   <212> PRT
   <213> Homo sapiens
<400> 52
   Thr Asp Gly Lys Asp Tyr Ile Ala Leu Asn Glu Asp Leu Ser Ser Trp
   1               5                   10                  15
   Thr
<210> 53
   <211> 14
   <212> PRT
   <213> Homo sapiens
<400> 53
   Leu Ala Val Val Lys Ser Ile Arg Ser Ile Pro Tyr Leu Ala
   1 5 10
<210> 54
   <211> 15
   <212> PRT
   <213> Homo sapiens
<400> 54
   Leu Leu Ala Val Val Lys Ser Ile Arg Ser Ile Pro Tyr Leu Ala
   1 5 10 15
<210> 55
   <211> 13
   <212> PRT
   <213> Homo sapiens
<400> 55
   Val Ala Asp Lys Ile Gln Leu Ile Asn Met Leu Asp Lys
   1 5 10
<210> 56
   <211> 14
   <212> PRT
   <213> Homo sapiens
<400> 56
   Asp Gln Val Ile Lys Val Phe Asn Asp Met Lys Val Arg Lys
   1 5 10
<210> 57
   <211> 15
   <212> PRT
   <213> Homo sapiens
<400> 57
   Leu Arg Thr Ile Asp Val Phe Asp Gly Asn Ser Gly Lys Met Met
   1 5 10 15
<210> 58
   <211> 15
   <212> PRT
   <213> Homo sapiens
<400> 58
   Gly Lys Val Asp Ile Val Ala Ile Asn Asp Pro Phe Ile Asp Leu
   1               5                   10                  15
<210> 59
   <211> 14
   <212> PRT
   <213> Homo sapiens
<400> 59
   Asp Asp Ile Arg Gly Ile Gln Ser Leu Tyr Gly Asp Pro Lys
   1               5                   10
<210> 60
   <211> 15
   <212> PRT
   <213> Homo sapiens
<400> 60
   Ala Asp Asp Ile Arg Gly Ile Gln Ser Leu Tyr Gly Asp Pro Lys
   1               5                   10                  15
<210> 61
   <211> 15
   <212> PRT
   <213> Homo sapiens
<400> 61
   Ser Ser Asn Val Val His Leu Ile Lys Asn Ala Tyr Asn Lys Leu
   1               5                   10                  15
<210> 62
   <211> 16
   <212> PRT
   <213> Homo sapiens
<400> 62
   Leu Asn Gln Glu Leu Arg Ala Asp Gly Thr Val Asn Gln Ile Glu Gly
   1               5                   10                  15
<210> 63
   <211> 15
   <212> PRT
   <213> Homo sapiens
<400> 63
   Gly Pro Leu Lys Phe Leu His Gln Asp Ile Asp Ser Gly Gln Gly
   1 5 10 15
<210> 64
   <211> 17
   <212> PRT
   <213> Homo sapiens
<400> 64
   Gly Pro Leu Lys Phe Leu His Gln Asp Ile Asp Ser Gly Gln Gly Ile
   1 5 10 15
   Arg
<210> 65
   <211> 17
   <212> PRT
   <213> Homo sapiens
<400> 65
   Val Lys Ser Glu Ile Ile Pro Met Phe Ser Asn Leu Ala Ser Asp Glu
   1 5 10 15
   Gln
<210> 66
   <211> 16
   <212> PRT
   <213> Homo sapiens
<400> 66
   Gly Ser His Ser Met Arg Tyr Phe His Thr Ala Met Ser Arg Pro Gly
   1 5 10 15
<210> 67
   <211> 17
   <212> PRT
   <213> Homo sapiens
<400> 67
   Asp Gly Lys Asp Tyr Ile Ala Leu Asn Glu Asp Leu Ser Ser Trp Thr
   1 5 10 15
   Ala
<210> 68
   <211> 16
   <212> PRT
   <213> Homo sapiens
<400> 68
   Lys Arg Lys Thr Val Thr Ala Met Asp Val Val Tyr Ala Leu Lys Arg
   1 5 10 15
<210> 69
   <211> 15
   <212> PRT
   <213> Homo sapiens
<400> 69
   Lys Arg Lys Thr Val Thr Ala Met Asp Val Val Tyr Ala Leu Lys
   1 5 10 15
<210> 70
   <211> 17
   <212> PRT
   <213> Homo sapiens
<400> 70
   Ala Lys Arg Lys Thr Val Thr Ala Met Asp Val Val Tyr Ala Leu Lys
   1 5 10 15
   Arg
<210> 71
   <211> 15
   <212> PRT
   <213> Homo sapiens
<400> 71
   Ser Pro Lys Tyr Ile Lys Met Phe Val Leu Asp Glu Ala Asp Glu
   1 5 10 15
<210> 72
   <211> 16
   <212> PRT
   <213> Homo sapiens
<400> 72
   Ser Pro Lys Tyr Ile Lys Met Phe Val Leu Asp Glu Ala Asp Glu Met
   1 5 10 15
<210> 73
   <211> 18
   <212> PRT
   <213> Homo sapiens
<400> 73
   Gly Ser Ser Arg Val Leu Ile Thr Thr Asp Leu Leu Ala Arg Gly Ile
   1 5 10 15
   Asp Val
<210> 74
   <211> 16
   <212> PRT
   <213> Homo sapiens
<400> 74
   Thr Ala Gln Val Ile Ile Leu Asn His Pro Gly Gln Ile Ser Ala Gly
   1 5 10 15
<210> 75
   <211> 13
   <212> PRT
   <213> Homo sapiens
<400> 75
   Val Ile Ile Leu Asn His Pro Gly Gln Ile Ser Ala Gly
   1 5 10
<210> 76
   <211> 17
   <212> PRT
   <213> Homo sapiens
<400> 76
   Val Tyr Lys Val Leu Lys Gln Val His Pro Asp Thr Gly Ile Ser Ser
   1               5                   10                  15
   Lys
<210> 77
   <211> 14
   <212> PRT
   <213> Homo sapiens
<400> 77
   Lys Val Leu Lys Gln Val His Pro Asp Thr Gly Ile Ser Ser
   1               5                   10
<210> 78
   <211> 15
   <212> PRT
   <213> Homo sapiens
<400> 78
   Lys Val Leu Lys Gln Val His Pro Asp Thr Gly Ile Ser Ser Lys
   1               5                   10                  15
<210> 79
   <211> 14
   <212> PRT
   <213> Homo sapiens
<400> 79
   Lys Ser Lys Ile Glu Asp Ile Arg Ala Glu Gln Glu Arg Glu
   1               5                   10
<210> 80
   <211> 13
   <212> PRT
   <213> Homo sapiens
<400> 80
   Lys Ser Lys Ile Glu Asp Ile Arg Ala Glu Gln Glu Arg
   1 5 10
<210> 81
   <211> 16
   <212> PRT
   <213> Homo sapiens
<400> 81
   His Asn Ser Leu Ile Ala Ser Ile Leu Asp Pro Tyr Ser Asn Ala Phe
   1 5 10 15
<210> 82
   <211> 15
   <212> PRT
   <213> Homo sapiens
<400> 82
   Thr Thr Ala Tyr Phe Leu Tyr Gln Gln Gln Gly Arg Leu Asp Lys
   1 5 10 15
<210> 83
   <211> 17
   <212> PRT
   <213> Homo sapiens
<400> 83
   Asn Arg Gln Val Asn Lys Lys Leu Asn Lys Thr Asp Leu Pro Lys Leu
   1 5 10 15
   Leu
<210> 84
   <211> 17
   <212> PRT
   <213> Homo sapiens
<400> 84
   Ala Glu Phe Leu Leu His Met Leu Lys Asn Ala Glu Ser Asn Ala Glu
   1 5 10 15
   Leu
<210> 85
   <211> 15
   <212> PRT
   <213> Homo sapiens
<400> 85
   Ser Pro Lys Tyr Ile Lys Met Phe Val Leu Asp Glu Ala Asp Glu
   1 5 10 15
<210> 86
   <211> 18
   <212> PRT
   <213> Influenza B virus
<400> 86
   Pro Gly Lys Thr Gly Thr Ile Val Tyr Gln Arg Gly Ile Leu Leu Pro
   1 5 10 15
   Gln Lys
<210> 87
   <211> 18
   <212> PRT
   <213> Influenza B virus
<400> 87
   Lys Pro Gly Lys Thr Gly Thr Ile Val Tyr Gln Arg Gly Ile Leu Leu
   1 5 10 15
   Pro Gln
<210> 88
   <211> 19
   <212> PRT
   <213> Influenza B virus
<400> 88
   Lys Pro Gly Lys Thr Gly Thr Ile Val Tyr Gln Arg Gly Ile Leu Leu
   1 5 10 15
   Pro Gln Lys
<210> 89
   <211> 20
   <212> PRT
   <213> Influenza B virus
<400> 89
   Lys Pro Gly Lys Thr Gly Thr Ile Val Tyr Gln Arg Gly Ile Leu Leu
   1               5                   10                  15
   Pro Gln Lys Val
               20
<210> 90
   <211> 346
   <212> PRT
   <213> Influenza B virus
<400> 90
   Asp Arg Ile Cys Thr Gly Ile Thr Ser Ser Asn Ser Pro His Val Val
   1               5                   10                  15
   Lys Thr Ala Thr Gln Gly Glu Val Asn Val Thr Gly Val Ile Pro Leu
               20                  25                  30
   Thr Thr Thr Pro Thr Lys Ser His Phe Ala Asn Leu Lys Gly Thr Lys
           35                  40                  45
   Thr Arg Gly Lys Leu Cys Pro Thr Cys Leu Asn Cys Thr Asp Leu Asp
       50                  55                  60
   Val Ala Leu Gly Arg Pro Met Cys Val Gly Val Thr Pro Ser Ala Lys
   65                  70                  75                  80
   Ala Ser Ile Leu His Glu Val Arg Pro Val Thr Ser Gly Cys Phe Pro
                   85                  90                  95
   Ile Met His Asp Arg Thr Lys Ile Arg Gln Leu Pro Asn Leu Leu Arg
               100                 105                 110
   Gly Tyr Glu Lys Ile Arg Leu Ser Thr Gln Asn Val Ile Asn Ala Glu
           115                 120                 125
   Lys Ala Pro Gly Gly Pro Tyr Arg Leu Gly Thr Ser Gly Ser Cys Pro
       130                 135                 140
   Asn Ala Thr Ser Arg Ser Gly Phe Phe Ala Thr Met Ala Trp Ala Val
   145                 150                 155                 160
   Pro Lys Asp Asn Asn Lys Thr Ala Thr Asn Pro Leu Thr Val Glu Val
                   165                 170                 175
   Pro His Ile Cys Thr Lys Glu Glu Asp Gln Ile Thr Val Trp Gly Phe
               180                 185                 190
   His Ser Asp Asn Lys Thr Gln Met Lys Asn Leu Tyr Gly Asp Ser Asn
           195                 200                 205
   Pro Gln Lys Phe Thr Ser Ser Ala Asn Gly Val Thr Thr His Tyr Val
       210                 215                 220
   Ser Gln Ile Gly Gly Phe Pro Asp Gln Thr Glu Asp Gly Gly Leu Pro
   225                 230                 235                 240
   Gln Ser Gly Arg Ile Val Val Asp Tyr Met Val Gln Lys Pro Gly Lys
                   245                 250                 255
   Thr Gly Thr Ile Val Tyr Gln Arg Gly Ile Leu Leu Pro Gln Lys Val
               260                 265                 270
   Trp Cys Ala Ser Gly Arg Ser Lys Val Ile Lys Gly Ser Leu Pro Leu
           275                 280                 285
   Ile Gly Glu Ala Asp Cys Leu His Glu Lys Tyr Gly Gly Leu Asn Lys
       290                 295                 300
   Ser Lys Pro Tyr Tyr Thr Gly Glu His Ala Lys Ala Ile Gly Asn Cys
   305                 310                 315                 320
   Pro Ile Trp Val Lys Thr Pro Leu Lys Leu Ala Asn Gly Thr Lys Tyr
                   325                 330                 335
   Arg Pro Pro Ala Lys Leu Leu Lys Glu Arg
               340                 345

## Description

The present invention relates to methods useful for isolating antigenic peptides from a limited quantity of cells or bodily fluid from a mammalian organism in an amount sufficient to determine their sequence and identity. Therefore this invention relates to methods for identifying novel disease-associated antigens, e.g. tumor antigens and antigens involved in autoimmune diseases, to be utilized for diagnostic or therapeutic purposes. The methods of the present invention can also be utilized for controlling the quality of vaccines. More specifically, the methods of the invention can be used for determining the sequence of antigenic peptides presented via peptide receptors of dendritic cells which are the most important antigen presenting cells of the body and valuable tools for vaccination.

Pathological conditions, such as infectious diseases, autoimmune disorders or cancer, can be distinguished from healthy conditions by the expression of disease-specific molecules. In particular, proteins which are newly expressed, mutated or aberrantly expressed, can be utilized as markers for the respective malignancy.

A potent class of markers serving as both diagnostic and therapeutic tools are protein fragments or peptides bound to molecules of the major histocompatibility complex (MHC). In humans, MHC molecules are termed human leukocyte antigens (HLA). HLA-associated peptides are short, encompassing 9-25 amino acids (Kropshofer, H. & Vogt, A.B., Immunol Today 18 (1997) 77-82). On the one hand, these peptides are derived from self-proteins in order to establish self-tolerance. On the other hand, HLA-associated peptides are derived from foreign proteins of viral, fungal or bacterial origin in order to fight foreign invaders. Through activation of specialized immune cells, named T lymphocytes (short: T cells), HLA-peptide complexes are indispensable for mounting a cellular or humoral immune response. Particular self-peptides, denoted autoantigenic peptides, are erroneously recognized by autoaggressive T cells giving rise to autoimmune diseases. Conversely, the lack ofT cell recognition of self-peptides derived from tumor-specific antigens, contributes to immune evasion and progressive growth of tumors (Boon, T. et al., Ann Rev Immunol. 12 (1994) 337-265). Hence, increasing our knowledge about disease-associated marker peptides would be of considerable importance in tumor immunology and autoimmunity.

With regard to their function, two classes of MHC-peptide complexes can be distinguished (Germain, R., Cell 76 (1994) 287-299): (i) MHC class I-peptide complexes can be expressed by almost all nucleated cells in order to attract CD8+ cytotoxic T cells which lyse infected cells or tumor cells, (ii) MHC class II-peptide complexes are constitutively expressed only on so-called antigen presenting cells (APCs), such as B lymphocytes, macrophages or dendritic cells (DCs). In particular, DCs have the capacity to prime CD4+ T helper cells (Banchereau, J. & Steinman, R.M., Nature 392 (1998) 245-254). Moreover, DCs can be licensed to optimally activate cytotoxic CD8+ T cells: this is accomplished through prior interaction of their MHC class II-peptide complexes with CD4+ T helper cells (Ridge, T. et al., Nature 393 (1998) 474-478). Thus, peptides presented by MHC class II molecules on DCs play a superior role in the pathogenesis of diseases involving T cell-driven immune responses.

The apparent role of DCs in initiating immune responses has stimulated attempts to exploit DCs as vaccines, in particular against cancer (Dallal, R.M. & Lotze, M.T., Curr Opinion Immunol 12 (2000) 583-588). A key advance was the invention of techniques for differentiation of DCs in vitro from different sources including peripheral blood, e.g. adherent monocytes, or bone marrow-derived CD34+ stem-cell precursors. DCs differentiated and activated in vitro can be used for vaccination of cancer patients after co-culture with tumor cell-derived antigens or by employing analogous techniques. Pilot dendritic cell vaccination studies have successfully induced specific anticancer responses including clinical responses (Timmermann, J.M. & Levy, R., Ann Rev Medicine 50 (1999) 507-529; Nestle, F.O., et al., Nature Medicine 7 (2001) 761-765).

DC-based cancer cell vaccines comprise DCs pulsed with normal or gene-modified cancer cells, cancer cell lysates, cancer cells fused to DCs or cancer cell-derived heat shock protein- (Hsp-) peptide complexes. The rationale behind the latter technique is that Hsps derived from tumor cells carry tumor-specific peptides which are efficiently transferred onto MHC molecules of DCs. These DCs finally prime cytotoxic T cells with anti-tumor reactivity which leads to the eradication of tumors in mice (Srivastava, P.K., et al., PNAS 83 (1986) 3407-3411; Suto, R., et al., Science 269 (1995) 1585-1588; Binder, R.J. et al, Nature Immunol. 1 (2000) 151-162).

The advantage of all these approaches is that no knowledge about the identity of tumor antigens is necessary. The disadvantage is that the identity of tumor markers remains unknown and the copy number of individual HLA-bound tumor peptides is often too low to induce long-lasting anti-tumor T cell responses.

Vaccines based on the identification of cancer antigens include DCs primed with naked DNA, recombinant adeno- or vaccinia viruses, natural or recombinant proteins purified from the respective tumor cells or synthetic analogs of tumor peptides. The advantage of pulsing DCs with antigenic tumor peptides rather than with genetic or protein precursors is that peptides can be loaded directly onto MHC molecules of DCs without further processing.

During the past decade, numerous peptides derived from tumor marker proteins and restricted by MHC class I molecules have been identified. They are grouped into four categories: cancer-testes antigens, melanoma-melanocyte differentiation antigens, mutated antigens and non-mutated shared antigens over-expressed on tumors. In several clinical pilot vaccination studies, DCs from melanoma patients were pulsed with cocktails of melanoma peptides which, as yet, were exclusively HLA class I-restricted (Nestle, F.O. et al., Nature Medicine 4 (1998) 328-332; Thurner, B. et al., J Exp Med 190 (1999) 1669-1678). However, there is increasing evidence that the efficacy and longevity of cytotoxic T cell responses against tumors can be increased by the involvement of MHC class II - restricted helper T cells. Hence, an improved vaccination method would foresee the combinatorial use of MHC class II associated tumor peptides in addition to MHC class I antigens.

Knowledge of MHC class II-restricted cancer antigens recognized by CD4+ T helper cells lags behind the identification of class I-restricted antigens (Wang, R.-F., Trends in Immunol 22 (2001) 269-276). One reason is that transfection of cDNA libraries from tumor cells into target cells and then using anti-tumor T cells to identify the appropriate transfectants and antigenic epitopes - a method successfully employed with MHC class 1 molecules - is not effective because the encoded proteins do not travel to the MHC class II pathway in APCs.

An innovative alternative is to use autologous DCs pulsed with tumor cells or particular tumor marker proteins and sequence the peptides associated to MHC or Hsp molecules on DCs. This approach, however, has not been employed so far, since DCs are non-dividing in vitro and only available in very small amounts from peripheral blood or bone marrow. Moreover, peptide purification and sequencing techniques were by far too insensitive, as yet, to directly identify disease-associated peptides by this or any other approach.

The same limitations are evident in the context of autoimmune diseases, such as rheumatoid arthritis (RA). RA patients suffer from systemic destruction of their joint tissue, which is mediated by auto-aggressive T lymphocytes and auto-antibodies. The presence of both auto-reactive T cells and antibodies rely on the presentation of MHC class II-restricted peptide antigen. In accordance with that, HLA-DR molecules, particularly the genes DRB1*0401 and DRB1*0404 in people of European descent, revealed to be major risk factors and confer increased susceptibility to RA (Marrack P et al. Nat. Med., 2002, 7: 899-905). Several candidate auto-antigens, such as collagen type II, fiaggrin, IgG and cartilage glycoprotein gp39, have been proposed. The corresponding autoantigenic peptides have been elucidated by indirect means only, e.g. the capacity to activate T cell clones present in serum or synovial fluid in RA patients. Recently, it became clear that none of the major CD4+ T cell clones accumulating in inflamed synovia of joints of RA patients recognizes the respective epitopes (Kotzin BL et al., PNAS (2000), 97, 291-296). A likely rationale for this is that it was not possible, as yet, to sequence HLA-DR-associated peptides from synovial tissue in RA, so that direct identification of autoantigenic peptides is still missing.

Hence, similar as in the tumor field, establishing a methodology that allows sequencing of naturally processed MHC peptides in the femtomolar range is a major need.

Kropshofer, H., et al., Science 270 (1995) 1357-1359, describe a method for isolating antigenic peptides in the femtomolar range.

The present invention provides methods for isolating and identifying femtomolar amounts of peptide antigens presented by 0.2 to 3 µg MHO class II molecules isolated from an organism or from cells derived from an organism. Said methods concern immune monitoring of diseases, e.g. autoimmune diseases, the design of individualized peptide vaccines for the treatment of diseases, e.g. of cancer and the quality control of vaccines e.g. those based on dendritic cells. The methods of the invention have the advantage that the identity of bound and/or presented antigenic peptides can be elucidated from very small amounts of bodily fluids or cells isolated from an mammalian organism.

The described methods ensure that the antigenic peptides isolated and identified are those that are bound and/or presented by peptide receptors in vivo or are those that are naturally-processed and presented by APCs, preferably DCs in vitro.

### Method

The present invention provides a method for isolating disease-associated antigenic peptides in femtomolar amounts allowing their identification which method comprises providing complexes of peptide receptors with antigenic peptides from a mammalian organism in an amount of 0.2 to 3 µg. This quantity equals to the amount of material which is normally available from biopsies or bodily fluids of patients or healthy donors. The lowest amount of material necessary in the prior art is about 200 µg MHC class II molecules derived from an unlimited source (inbred mice) (Dongre AR et al., EJI 2001, 31, 1485-94). This is about two orders of magnitude more material than available from human patient material.

The amount of tissue or bodily fluid necessary to obtain e.g. 100 ng MHC class II molecules depends on the number of cells that do express MHC class II and on the expression rate of MHC class II molecules: e.g. 100 ng of MHC class II are equivalent to about 2 x 10⁵ mature DCs or 5 to 10 x 10⁶ peripheral blood monocytes or about 5 x 10⁷ peripheral blood mononuclear cells which can be obtained from about 50 ml of blood.

The high sensitivity required for identifying MHC associated peptides is explained by the fact that each type of these peptide receptors, e.g. human MHC class II gene product HLA-DR1, carries about 500 to 1000 different antigenic peptides (Chicz RM et al., J Exp. Med. 1993, 178, 27-47; Chicz RM & Urban RG, Immunol. Today, 1993, 15: 155-160). However, most of the 500 to 1000 different peptides attain very low copy numbers and, therefore, are not very likely to play a physiological role. Especially in the MHC class II field, those peptides that are of immunological relevance e.g. those that activate helper T cells, attain moderate to high copy numbers (Latek RR & Unanue ER, Immunol. Rev. 1999, 172: 209-228). These peptides cover about 40 to 50% of the total amount of peptide material eluted from MHC class II molecules and equal to about 10 to 200 individual peptides.

Many MHC class II associated peptides are represented as a set of 2 to 5 C- and N-terminal truncation variants (Rudensky AY et al, Nature 1992, 359, 429-431; Chicz et al. Nature 1992, 358: 764-768) sharing a common core sequence of about 10 to 13 amino acids which is essential for recognition by the T cell receptor. These truncation / elongation variants constitute the same T cell epitope. This means that the number of different epitopes, which are of importance is actually smaller, ranging from about 5 to 70 different epitopes. Thus, the abundance of immunologically relevant epitopes ranges from 0.2% to 5%.

The method of the present invention consists of
(a) providing complexes of MHC II molecules with antigenic peptides isolated from cells, tissue samples or body fluid of a mammalian organism in an amount of 0.2 to 3 µg and
(b) sequestering the complexes by immunoprecipitation, wherein the complexes are coupled by MHCII specific antibodies to beads,
c) washing the bound complexes of MHCII molecules with antigenic peptides with water or low salt buffer, wherein the low salt buffer is a Tris, phosphate or acetate buffer in a concentration range of 0.5 to 10mM and
d) eluting the associated antigenic peptides from the MHC II molecules.

### Origin of the peptides

The antigenic peptides of the present invention are peptides which are associated with peptide receptors from tissue or body fluids or cells of a mammalian organism or from antigen presenting cells derived from a mammalian organism. They may be bound to transmembrane peptide receptors comprising MHC II molecules presenting the antigenic peptides at the cell surface to T cells of the immune system. The antigenic peptides may also be bound to intra- or extracellular MHC molecules.

The antigenic peptides comprise self antigens, tumor antigens, autoantigens, viral, bacterial, and parasitic antigens. Some antigenic peptides may induce tolerance. Other antigenic peptides may elicit an immune response and are therefore immunogenic peptides. The antigenic peptides of the present invention may be naturally processed antigenic peptides that means they are generated from antigenic proteins by the proteolytic system of the respective cell and loaded onto peptide receptors. The antigenic peptides may also be non-naturally processed synthetic or recombinant antigenic peptides which may have been administered to an organism where they have been loaded onto peptide receptors without further processing or they may have been contacted with cells expressing peptide receptors in cell culture or with isolated peptide receptors in vitro.

Therefore, the methods of the present invention comprise antigenic peptides, which are naturally-processed antigenic peptides, as well as antigenic peptides which are synthetic or recombinant antigenic peptides.

As all these peptide receptors are able to accommodate a broad variety of peptide ligands (see above), each single peptide whose sequence has to be determined is represented in only femtomolar amounts. 1 µg MHC class II (16 pmol) may carry dominant peptide species, with each single peptide attaining an occupancy of 0.1 - 2%, which equals to about 16 - 320 femtomoles. The methods of the present invention allow the isolation of these femtomolar amounts of antigenic peptides from 0.1 to 5 µg of peptide receptors loaded with peptides and their subsequent sequencing.

Origin of the peptide receptors

In a further embodiment, the methods of the present invention relate to peptide receptors comprising all proteins binding antigenic peptides, consisting of MHC class II molecules.

Furthermore, complexes of peptide receptors with antigenic peptides may be isolated from diverse body fluids of an organism, e.g. of blood, serum, ascites, synovial fluid, from tissue samples of an organism, e.g. tumor biopsies, or from cells isolated from an organism.

Complexes of peptide receptors with antigenic peptides may be isolated from a mammalian organism, preferably from a human organism.

The peptide receptors are isolated from an organism in an amount of 0.2 to 3 µg.

### Origin of the cellular material

The methods of the present invention encompass cells expressing MHC II molecules comprising B cells, macrophages, dendritic cells, monocytes, thymic epithelial cells, microglial cells activated T cells and endothelial and epithelial cells after induction with pro-inflammatory cytokines e.g. IFNgamma. The cells expressing MHC II molecules are also referred to as antigen presenting cells (APCs) (Unanue, E.R.. Macrophages, antigen presenting cells and the phenomena of antigen handling and presentation. In: Fundamental Immunology, 2nd edition (editor Paul, W.E) New York, Raven Press, 1989).

### Solubilization of peptides receptors from cells

For the purification of peptide receptor-peptide complexes from cells or tissue, the membranes of the cells or tissue have to be solubilized. Cell lysis may be carried out with methods known in the art, e.g. freeze-and-thaw cycles and the use of detergents, and combinations thereof. Preferred lysis methods are solubilization using detergents, preferably TX-100, NP40, n-octylglucoside, Zwittergent, Lubrol, CHAPS, most preferably TX-100 or Zwittergent 3-12. Cell debris and nuclei have to be removed from cell lysates containing the solubilized receptor-peptide complexes by centrifugation. Therefore, in a further embodiment of the present invention, the complexes of peptide receptors with antigenic peptides are isolated from the cells with methods comprising solubilization with a detergent.

### Nano-scale purification of MHC-peptide complexes

Furthermore, the invention provides the purification of the MHC class II peptide complexes from cell lysates by immunoprecipitation.

For the immunoprecipitation, antibodies specific for MHC class II molecules and suitable for these methods are used. The specific antibodies are preferably monoclonal antibodies, and are covalently or non-covalently e.g. via Protein A, coupled to beads, e.g. sepharose or agarose beads. A selection of the broad panel of anti-HLA antibodies used in the prior art comprises:
anti-HLA-DR antibodies: L243, TU36, DA6.147, preferably L243; anti-HLA-DQ antibodies: SPVL3, TU22, TU169, preferably TU22 and TU169; anti-HLA-DP antibody B7/21 and anti-HLA-A,B,C antibodies W6/32 and B9.12.

Monoclonal antibodies specific for different MHC class II molecules may be commercially obtained (e.g. Pharmingen, Dianova) or purified from the supernatant of the respective hybridoma cells using Protein A- or Protein G- affinity chromatography. Purified monoclonal antibodies may be coupled by various methods known in the art, preferably by covalently coupling antibody amino groups to CNBr-activated sepharose.

Immunoisolation of MHC molecules may be performed by incubating the antibody-beads with the cell lysate under rotation for several hours. Washing of the antibody-beads may be performed in eppendorf tubes. The efficacy of the immunoprecipitation may be analysed by SDS-PAGE and western blotting using antibodies recognizing denatured MHC molecules (anti-HLA-DRalpha: 1B5; anti-HLA class I: HC10 or HCA2).

### Elution and fractionation of peptide receptor-associated peptides

By eluting the peptides from the receptor molecules, a complex mixture of naturally processed peptides derived from the source of potential antigen and from polypeptides of intra- or extracellular origin, is obtained. Only after elution, peptides can be fractionated and subjected to sequence analysis.

The antigenic peptides in the methods of the present invention may be eluted by a variety of methods known in the art, preferably by using diluted acid, e.g., diluted acetonitrile (Jardetzky TS et al., Nature 1991 353, 326-329), diluted acetic acid and heating (Rudensky AY et al., Nature 1991, 353, 622-626; Chicz RM et al., Nature 1992, 358, 764-768) or diluted trifluoro acetic acid at 37°C (Kropshofer H et al., J Exp Med 1992, 175, 1799-1803). Most preferably, the peptides are eluted at 37°C with diluted trifluoro acetic acid.

The sequestered peptide receptor-peptide complexes are washed with water or low salt buffer before elution in order to remove residual detergent contaminants. The low salt buffer is a Tris, phosphate or acetate buffer in a concentration range of 0.5 - 10 mM, preferably in a concentration of 0.5 mM. In a more preferred embodiment, the peptide receptor-peptide complexes are washed with ultrapure water (sequencing grade) conventionally used for HPLC analysis, preferably with ultrapure (sequencing grade) water from MERCK. The washing step may be carried out by ultrafiltration. The ultrafiltration may be carried out in an ultrafiltration tube with a cut-off of 30 kD, 20 kD, 10 kD or 5 kD, preferably of 30 kD and a tube volume of 0.5 - 1.0 ml ("Ultrafree" tubes; Millipore). The washing in the ultrafiltration tube may be carried out 4 to 12 times, preferably 6 to 10 times, with a volume of 10 to 20 times the volume of the beads carrying the receptor-peptide complexes, preferably with a volume of 15 times the beads. The eluted peptides may be separated from the remaining peptide receptor molecules using the same ultrafiltration tube. The eluted peptides may then be lyophilized.

### Peptide sequence analysis by liquid chromatography-mass spectrometry (LC-MS)

In a further embodiment of the present invention, the isolated antigenic peptides are fractionated, sequenced and identified. By sequencing it is understood that the amino acid sequence of the individual peptides in the mixture of isolated antigenic peptides is elucidated by methods adequate to sequence femtomolar amounts of peptides. By identifying it is understood that it is established from which proteins or polypeptides the antigenic peptides are derived and which sequence they constitute within these proteins or polypeptides.

In a first step, the complex mixture of eluted peptides may be fractionated by one of a variety of possible chromatographic methods, e.g. by reversed phase, anion exchange, cation exchange chromatography or a combination thereof. Preferably, the separation is performed by C18-reverse phase chromatography or by reversed-phase / cation exchange two-dimensional HPLC, denoted as MudPit (Washburn MP et al., Nat Biotechnol., (2001), 19, 242-247).

The fractionation is done in a HPLC mode utilizing fused-silica micro-capillary columns which are either connected to a nano-flow electrospray source of a mass spectrometer or to a micro-fractionation device which spots the fractions onto a plate for MALDI analysis.

A variety of mass spectrometric techniques are suitable, preferably MALDI-post source decay (PSD) MS or electrospray ionization tandem mass spectrometry (ESI-MS), most preferably ion-trap ESI-MS.

The sequences of the individual peptides can be determined by means known in the art. Preferably, sequence analysis is performed by fragmentation of the peptides and computer-assisted interpretation of the fragment spectra using algorithms, e.g. MASCOT or SEQUEST. Both computer algorithms use protein and nucleotide sequence databases to perform cross-correlation analyses of experimental and theoretically generated tandem mass spectra. This allows automated high through put sequence analysis.

### Qualitative peptide analysis by MALDI mass spectrometry

For qualitative analysis of the whole peptide repertoire obtained upon elution, matrix-assisted laser desorption and ionization time-of-flight (MALDI-TOF) mass spectrometry may be carried out. Using settings that do not fragment the peptides, MALDI-TOF analysis provides a rough overview with regard to the complexity of the peptide mixture and the presence of dominant peptides.

### Quantitative peptide analysis

To estimate the quantity of single peptides eluted from peptide receptors, the run through of the micro-capillary column may be analyzed by a flow-through UV detector operated at a detection wave-length of 214 nm. For quantitation the peak areas of peptides to be analyzed are compared with peak areas of graded amounts of synthetic standard peptides.

### Strategy 1 (ex-vivo approach)

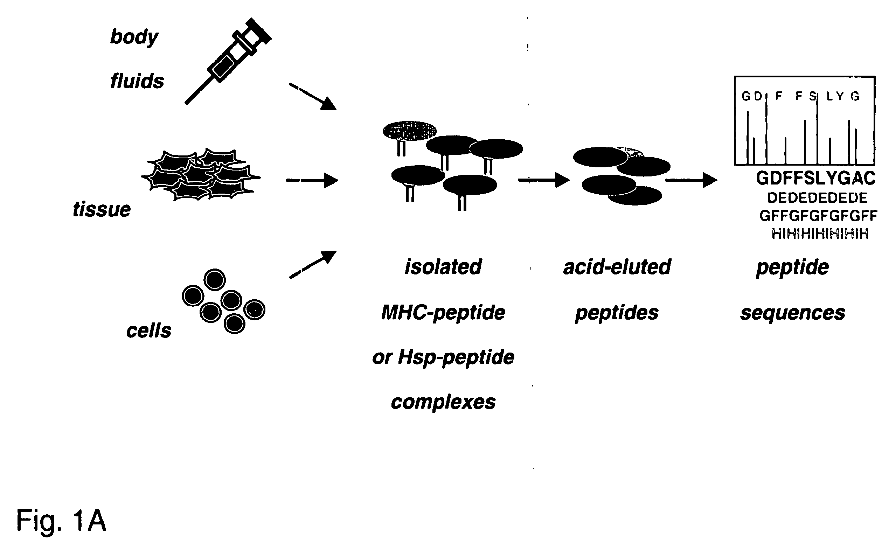

Strategy 1 of the present invention is used to isolate antigenic peptides which were loaded onto peptide receptors inside an organism (ex vivo approach, Fig. 1A).

The present invention relates to a method for isolating and identifying MHC class II associated peptides in femtomolar amounts which method comprises providing 0.2 to 3 µg MHC class II-peptide complexes from a mammalian organism. This amount of peptide receptors equals to the amount of material which is normally available from biopsies or bodily fluids of patients or healthy donors, e.g. 100 ng of MHC class II-peptide complexes can be purified from about 5 x 10⁷ peripheral blood mononuclear cells isolated from about 50 ml of blood.

The MHC class II-peptide complexes may be purified from isolated cells e.g. blood monocytes, from a mixture of cells e.g. peripheral blood mononuclear cells, from tissue, e.g. tumor biopsies, or from body fluids e.g. ascites or synovial fluid.

The body fluids may contain MHC class II-peptide complexes bound to cells present in the body fluid, e.g. in synovial fluid, bound to vesicles present in the body fluid e.g. apoptotic vesicles or exosomes derived from cells (Denzer K et al., J Cell Science, 2000, 113, 3365-3374), or MHC class II-peptide complexes may be present in soluble form, due to shedding from the plasma membrane, e.g. soluble MHC class II molecules (Aultman D et al., Human Immunol., 1999, 60, 239-244).

MHC Class II-peptide complexes maybe isolated from diverse body fluids of an organism, e.g. of blood, serum, ascites, synovial fluid or from tissue samples of an organism, e.g. biopsies, excised primary or secondary tumors or from cells isolated from an organisms.

Complexes of peptide receptors with antigenic peptides may be isolated from cells, tissue or body fluids from a mammalian organism, preferably from a human organism.

### Strategy 2 (in vitro approach)

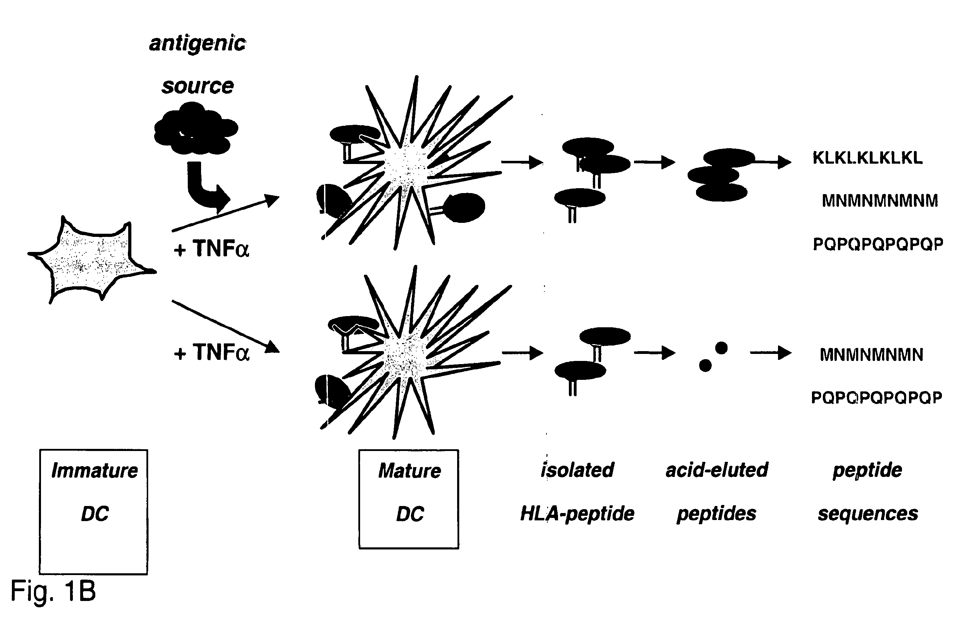

Strategy 2 of the present invention foresees isolation of antigenic peptides which have been loaded onto peptide receptors outside an organism, e.g. in cell culture (in vitro approach, Fig. 1B).

In a further embodiment the present invention relates to a method for isolating antigenic peptides in femtomolar amounts which method consists of (a) providing MHC class II expressing cells in a numer providing 0.2 to 3 µg MHC class II molecules, (b) contacting the cells with a source of potential antigen, (c) isolating MHC class II molecule-antigenic peptide complexes from the cells and (d) eluting the associated peptides from the MHC class II molecules.

The MHC class II expressing cells may be MHC II expressing cells (APCs). Preferably, APCs are dendritic cells, more preferably, the APCs are immature dendritic cells, most preferably, the APCs are immature dendritic cells generated from peripheral blood monocytes.

Dendritic cells may be generated from peripheral blood monocytes or from bone marrow-derived CD34+ stem cell-precursors. The peripheral blood mononuclear cells (PBMCs) may be isolated from blood samples by density gradient centrifugation. The monocytes may then be isolated from PBMCs by methods known in the art, e.g. by sorting with magnetic beads. The source of dendritic cells may be mammalian species, preferably humans. The monocytes may then be differentiated in cell culture to become immature dendritic cells. The differentiation state may be monitored by flow-cytometric analysis, e.g. using upregulation cell surface markers CD83, CD80, CD86, HLA-DR.

The amount of tissue or bodily fluid necessary to obtain e.g. 100 ng MHC class II molecules depends on the number of cells that do express MHC class II and on the expression rate of MHC class II molecules: e.g. 100 ng of MHC class II are equivalent to about 2 x 10⁵ mature DCs or 5 to 10 x 10⁶ peripheral blood monocytes or about 5 x 10⁷ peripheral blood mononuclear cells which can be obtained from about 50 ml of blood.

The APCs are then contacted with a source of potential antigen. The APCs, preferably the immature dendritic cells, are at the same time triggered to mature by methods known in the art, e.g. incubation with inflammatory cytokines, like TNF alpha or a mixture of TNF alpha, IL-6, IL1beta PGE2.

The source of potential antigen offered to the APCs may be selected from the group comprising tumor tissue, tumor cells, tumor cell lines, gene-modified tumor cell lines, a crude cellular lysate of these cells or cell lines, tumor cell-derived heat shock proteins, pathogens, known viral, bacterial and parasitic antigens, tissues subject to immune attack, known self antigens, autoantigens, body fluids or tissue biopsies from patients with tumor, autoimmune or infectious diseases, body fluids or tissue biopsies from healthy individuals as reference controls. Control APCs are treated equivalently except that they are not exposed to a source of potential antigen. The source of potential antigen may be derived from different mammalian species, preferably from human.

The APCs may be contacted with a source of potential antigen which is taken up by the APCs by receptor-mediated uptake or by fluid phase uptake and internalized. The cells may also be infected with a source of potential antigen, e.g., with a virus.

By eluting the peptides from the MHC molecules, a set of naturally processed peptides derived from the source of potential antigen as well as from polypeptides of intracellular or extracellular origin, is obtained.

### Epitope validation for MHC-associated peptides

The peptide sequences identified by the methods of the invention may be validated by one of several criteria, comprising MHC binding motif, MHC binding capacity and T cell recognition.

MHC binding motifs are common structural characteristics of peptides associated to a particular MHC molecule (allelic variant) which are necessary to form stable complexes with MHC molecules. Peptide ligands eluted from MHC class I molecules are relatively short, ranging from 8 to 11 amino acids. Moreover, 2 or 3 side chains of the peptide are relevant for binding. The position of the respective amino acid side chains varies with the HLA allele, most often two of these so-called "anchor" residues are located at positions 2 and 9. With respect to a particular anchor position, only 1 or 2 amino acids normally can function as anchor amino acids e.g. leucine or valine V at position 2 in the case of HLA-A2.

In the case of MHC class II molecules, the peptide length varies from 12 to 18 amino acids and even longer peptides can bind since both ends of the peptide binding groove are open. Most HLA class II molecules accommodate up to 4 anchor residues at relative positions P1, P4, P6 and P9 contained in a nonameric core region. This core region, however, can have variable distance from the N-terminus of the peptide. In the majority of cases, 2-4 N-terminal residues precede the core region. Hence, the P1 anchor residues is located at positions 3, 4 or 5 in most HLA class II associated peptides. Peptides eluted from HLA-DR class II molecules share a big hydrophobic P1 anchor, represented by tyrosine, phenylalanine, tryptophane, methionine, leucine, isoleucine or valine.

The position and the exact type of anchor residues constitute the peptide binding motif which is known for most of the frequently occurring HLA class II allelic products. A computer algorithm allowing motif validation in peptide sequences is "Tepitope", available by vaccinome (www.vaccinome.com).

The MHC binding capacity of the peptides identified by the methods of the present invention may be tested by methods known in the art using, for example, isolated MHC class II molecules and synthetic peptides with amino acid sequences identical to those identified by the method of the invention (Kropshofer H et al., J. Exp. Med. 1992; 175, 1799-1803; Vogt AB et al., J. Immunol. 1994; 153, 1665-1673; Sloan VS et al., Nature 1995; 375, 802-806). Alternatively, a cellular binding assay using MHC class II expressing cell lines and biotinylated peptides can be used to verify the identified epitope (Arndt SO et al., EMBO J., 2000; 19, 1241-1251)

In both assays, the relative binding capacity of a peptide is measured by determining the concentration necessary to reduce binding of a labelled reporter peptide by 50% (IC50). Peptide binding with a reasonable affinity to the relevant HLA class II molecules attains IC50 values not exceeding 10-fold the IC50 of established reference peptides.

The same binding assays can also be used to test the ability of peptides to bind to alternative class II MHC molecules, i.e., class II MHC molecules other than those from which they were eluted using the method of the invention.

T cell recognition may represent another epitope verification procedure involving testing of peptides identified by the methods of the invention for their ability to activate CD4+ T cell populations. CD4+ T cell are activated by peptides binding to MHC class II molecules I. Peptides with amino acid sequences either identical to those identified by the methods of the invention or corresponding to a core sequence derived from a nested group of peptides identified by the methods of the invention are synthesized. The synthetic peptides are then tested for their ability to activate CD4+ T cells from (a) test subjects expressing the MHC class II molecule of interest and having at least one symptom of the disease; and (b) control subjects expressing the MHC class II molecule of interest and having no symptoms of the disease. Additional control subjects can be those with symptoms of the disease and not expressing the MHC class II molecule of interest.

In some diseases (e.g., those with an autoimmune component) responsiveness in the CD4+ T cells of test subjects but not in CD4+ T cells of the control subjects described in (b) provides confirmatory evidence that the relevant peptide is an epitope that activates CD4+ T cells that can initiate, promote, or exacerbate the relevant disease. In other diseases (e.g., cancer or infectious diseases without an autoimmune component), a similar pattern of responsiveness and non-responsiveness to that described in the previous sentence would indicate that the relevant peptide is an epitope that activates CD4+ T cells that can mediate immunity to the disease or, at least, a decrease in the symptoms of the disease.

CD4+ T cell responses can be measured by a variety of in vitro methods known in the art. For example, whole peripheral blood mononuclear cells (PBMC) can be cultured with and without a candidate synthetic peptide and their proliferative responses measured by, e.g., incorporation of [3H]-thymidine into their DNA. That the proliferating

T cells are CD4+ T cells can be tested by either eliminating CD4+ T cells from the PBMC prior to assay or by adding inhibitory antibodies that bind to the CD4+ molecule on the T cells, thereby inhibiting proliferation of the latter. In both cases, the proliferative response will be inhibited only if CD4+ T cells are the proliferating cells. Alternatively, CD4+ T cells can be purified from PBMC and tested for proliferative responses to the peptides in the presence of APC expressing the appropriate MHC class II molecule. Such APCs can be B-lymphocytes, monocytes, macrophages, or dendritic cells, or whole PBMC. APCs can also be immortalized cell lines derived from B-lymphocytes, monocytes, macrophages, or dendritic cells. The APCs can endogenously express the MHC class II molecule of interest or they can express transfected polynucleotides encoding such molecules. In all cases the APCs can, prior to the assay, be rendered non-proliferative by treatment with, e.g., ionizing radiation or mitomycin-C.

As an alternative to measuring cell proliferation, cytokine production by the CD4+ T cells can be measured by procedures known to those in art. Cytokines include, without limitation, interleukin-2 (IL-2), interferon-gamma (IFN-gamma), interleukin-4 (IL-4), TNF-alpha, interleukin-6 (IL-6), interleukin-10 (IL-10), interleukin-12 (IL-12) or TGF-beta. Assays to measure them include, without limitation, ELISA, and bio-assays in which cells responsive to the relevant cytokine are tested for responsiveness (e.g., proliferation) in the presence of a test sample.

Alternatively, cytokine production by CD4+ lymphocytes can be directly visualized by intracellular immunofluorescence staining and flow cytometry.

### Applications

The methods of the present invention can be applied to identify peptides involved in the pathogenesis of a wide range of diseases, especially those in which susceptibility has been associated with expression of one or several particular MHC alleles or those where MHC-restricted T cell responses are lacking.

Candidate diseases include, without limitation, autoimmune diseases (e.g. rheumatoid arthritis (RA), type I diabetes, multiple sclerosis (MS), coeliac disease, myasthenia gravis (MG) and systemic lupus erythematosus (SLE)), cancer (e.g. melanoma, breast cancer, B cell lymphomas, prostate cancer, renal cancer) or infectious diseases (e.g. diseases caused by HIV, hepatitis C virus, measles virus, mycobacteria).

One aspect of the invention is a therapeutic purpose, wherein one or more of the identified peptides are used to vaccinate patients against cancer or infectious diseases. To this end, the relevant peptides may be directly administered to the patient, in an amount sufficient for the peptides to bind to the MHC molecules, and provoke activation ofT cells followed by T cell-mediated lysis of infected or cancer cells.

Alternatively, the relevant peptides may be utilized for the generation of vaccines based on DCs. In this case, autologous DCs derived from patients' monocytes may be pulsed with the relevant peptides or recombinant proteins containing the relevant peptide sequences. Particularly, in vaccination against tumors, a combination of MHC class I- and class II-associated tumor antigenic peptides may be used to pulse DCs. Similarly, nucleic acid molecules which encode the relevant peptides may be incorporated into a vector in order to transfect tumor cells. These transfected tumor cells may be fused with DCs. In any of these cases, DCs presenting the relevant peptides in context of the appropriate MHC molecules will be administered to a patient for triggering a T cell response.

A further therapeutic application of the peptides relates to the situation where identified peptides are autoantigens in the context of autoimmune diseases. In this case, complexes of the respective autoantigenic peptide and its restricting MHC class II molecules may be targeted by chimeric or humanized antibodies. This approach may lead to diminishment of autoantigenic MHC class II peptide complexes and, thus, to a decline in the number of autoaggressive CD4+ T helper cells which belong to one of the driving forces in autoimmunity.

Therefore, the present invention provides the use of the described methods for the design of individualized peptide vaccines for the treatment of diseases, preferably cancer.

Beyond that, the method of the invention can be exploited for several diagnostic purposes. First, peptides of the invention may be used as response markers to track the efficacy of a therapeutic regime. Essentially, one can determine the baseline value for the relevant peptide, e.g. an autoantigenic peptide in the synovial fluid of rheumatoid arthritis patients, using strategy 1 of the invention, administer a given therapeutic drug and then monitor levels of the autoantigenic peptide thereafter, observing changes in peptide levels as indicia of the efficacy of the regime.

Therefore, the present invention provides the use of the described methods for the control of the efficacy of a therapeutic treatment.

In the same manner MHC class II associated peptides derived from blood of patients suffering from autoimmune diseases (e.g. RA, type I diabetes, MS, coeliac disease, MG or SLE) may be used as response markers for therapeutic drugs against autoimmune diseases.

Likewise, autoantigenic peptides which are only found in certain stages or phases of an autoimmune disease may be utilized as stage-specific markers.

Therefore, the present invention provides the use of the described methods for immune monitoring of diseases, preferably of autoimmune diseases.

A further aspect of the invention is a method for controlling the quality of vaccines based on DCs. Autologous DCs used as vaccines against tumors are subjected to tumor antigens in order to load MHC molecules of DCs with appropriate tumor antigenic peptides. A prerequisite of a high-quality DC vaccine is a high copy number of tumor antigenic peptides bound to the relevant MHC molecules. Applying strategy 2 of this invention, the presence or absence of the relevant peptides can be tested prior to utilizing the respective DC preparations for vaccination. Similarly, the quality of vaccines based on other APCs, e.g. macrophages or B cells, could be determined.

Therefore, the present invention provides the use of the described methods for the quality control of vaccines.

Finally, the method of the invention not only allows the identification of MHC-class II associated peptides, but, at the same time, the protein wherefrom the peptide is derived. These proteins may be used as diagnostic markers in the very same manner as described above for the corresponding peptides.

Having now generally described this invention, the same will become better understood by reference to the specific examples, which are included herein for purpose of illustration only and are not intended to be limiting unless otherwise specified, in connection with the following figures.
Fig. 1A is a diagram showing an overview of the methodology following strategy 1 (direct approach): MHC class II-peptide complexes or Hsp-peptide complexes are isolated directly from tissue or bodily fluids thereby leading to the identification of naturally processed MHC class II or Hsp associated antigens presented in vivo.
Fig. 1B is a diagram showing an overview of the methodology following strategy 2 (indirect approach): Dendritic cells (DCs), the most specialized antigen presenting cells (APCs), are brought in contact with an antigenic source (e.g. bodily fluids) under optimal conditions for antigen uptake and antigen processing. As a control, DCs are cultured under the same conditions without contact with antigens. After maturation of DC antigen loaded MHC class II molecules are purified and the respective MHC class II associated antigenic peptides are isolated and identified.

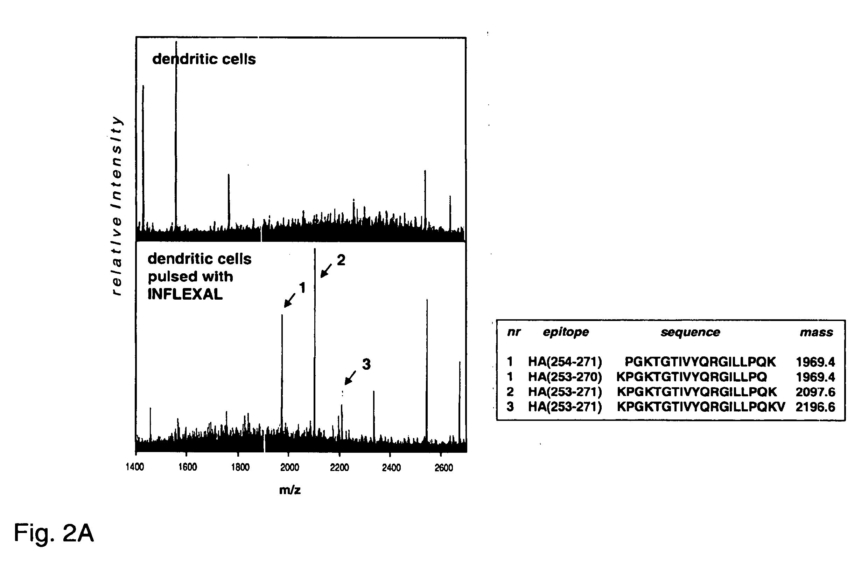

Fig. 2A illustrates strategy 2 and is a mass spectrometric analysis of HLA-DR bound peptides isolated from mature dendritic cells which were mock-treated (upper panel) or pulsed with the influenza vaccine Inflexal Berna V™, containing virosome-encapsulated recombinant hemagglutinin from Influenza virus (lower panel). The three major signals induced by treatment with Inflexal Berna V™ are marked by arrows and numbers.

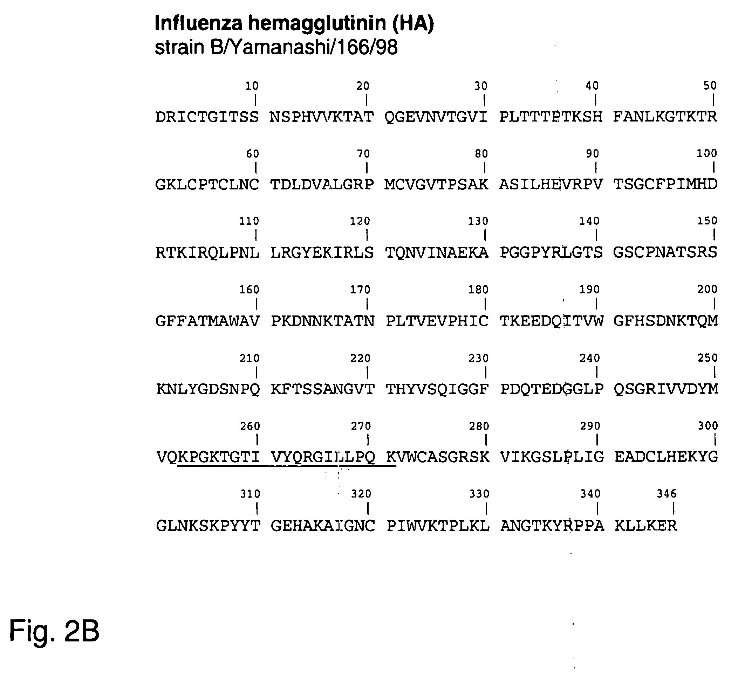

Fig. 2B shows the protein sequence (one-letter-code) of the influenza hemagglutinin protein from strain B / Yamanashi /166 / 98. The newly identified HLA-DR epitope (cf. Fig. 2A) is underlined.

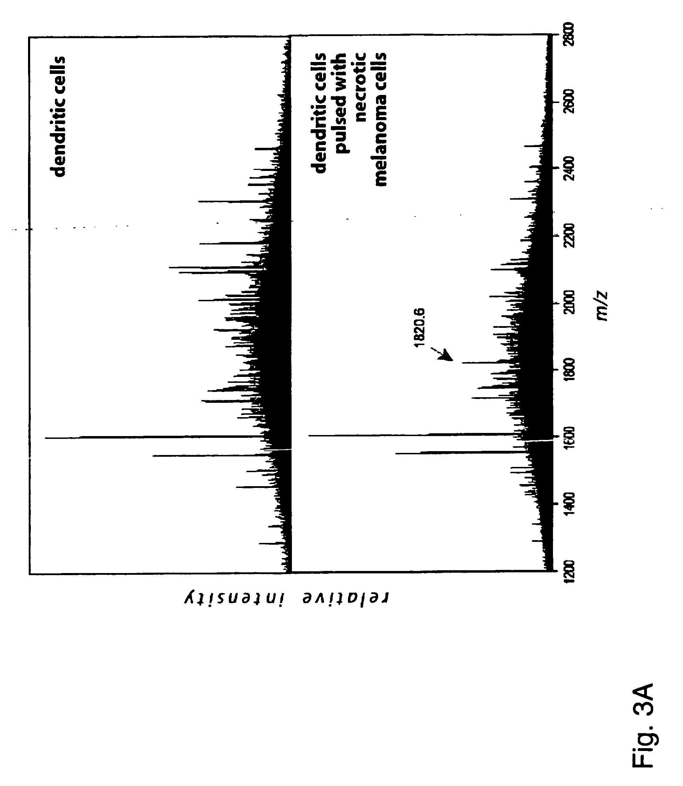

Fig. 3A contains a representative mass spectrometric analysis of the repertoire of HLA-DR bound peptides isolated from mature dendritic cells which have been mock treated (upper panel) or pulsed with the necrotic melanoma cell line UKRV-Mel-15a (lower panel). Marked is the peptide peak (M+H⁺)=1820.6 which became dominant in the profile upon contact with melanoma cells.

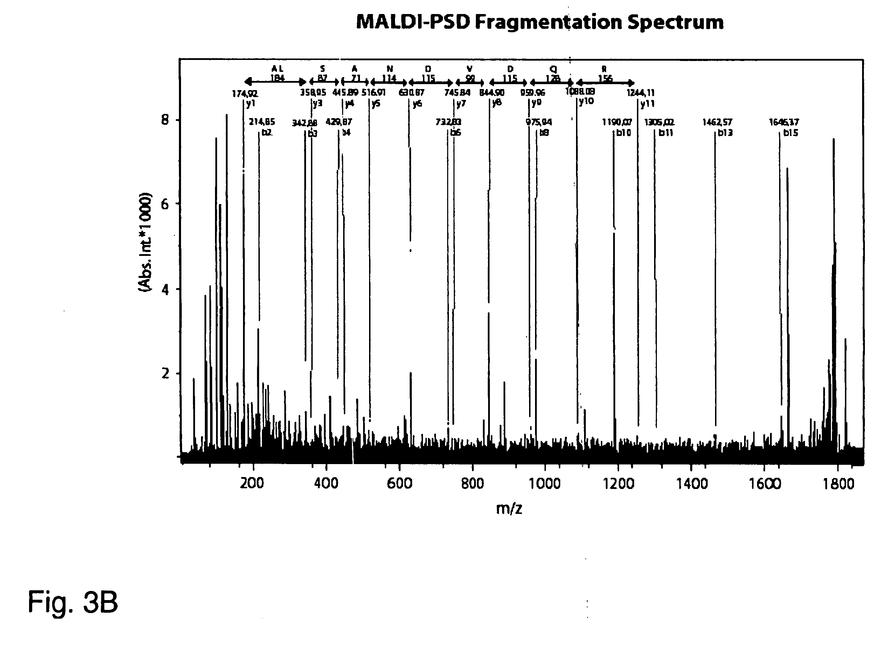

Figs. 3B shows the corresponding MALDI-PSD fragmentation spectrum of the peptide with the experimental mass (M+H+)=1820.6. This peptide was induced by necrotic melanoma cells (Fig. 3A). Data base search led to the identification of the vimentin epitope vimentin(202-217) (cf. Table 2).

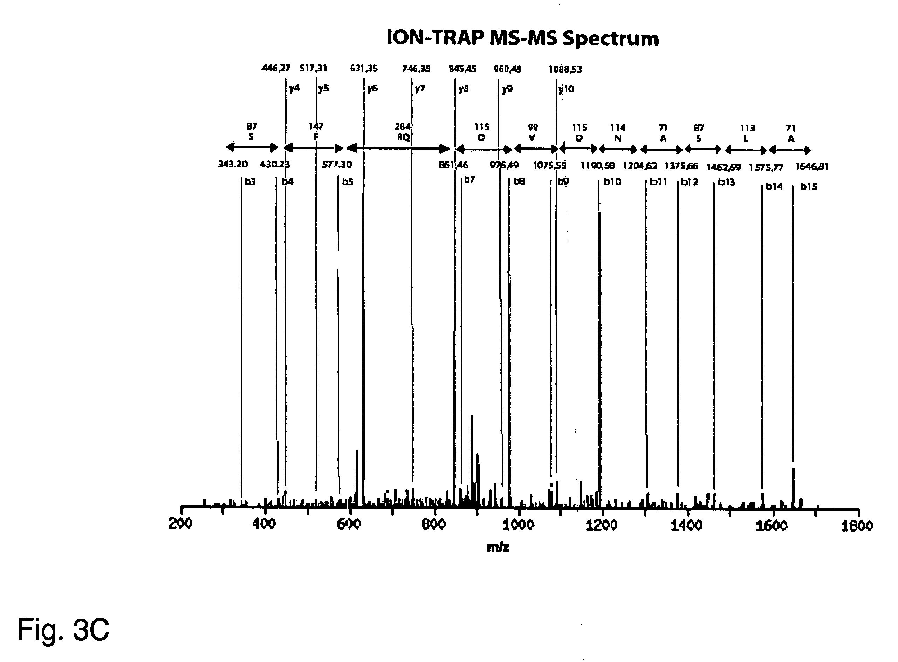

Figs. 3C shows an Ion trap MS-MS spectrum of a peptide with the experimental mass (M+H+)=1820.6. This peptide was induced by necrotic melanoma cells (Fig. 3A). Data base search led to the identification of the vimentin epitope vimentin(202-217) (cf. Table 2).

### Examples

The examples below are in connection with the figures described above and based on the methodology summarized in Figure 1A and Figure 1B and described in detail in the following. Commercially available reagents referred to in the examples were used according to manufacturer's instructions unless otherwise indicated.

### Methodology of the invention

### Cell lines and culture

The study was performed with human dendritic cells which were differentiated from monocytes, as described below. Monocytes were purified from human peripheral blood. In addition, the melanoma cell lines UKRV-Mel-15a and UKRV-Mel-20c (Eichmueller S et al., Exp Dermatol 2002; 11, 292-31) were utilized.

All cells were cultured in RPMI 1640 medium (short: RPMI) supplemented with 1 mM Pyruvat, 2 mM Glutamine and 10% heat-inactivated fetal calf serum (Gibco BRL, Rockville, MD).

### Isolation of peripheral blood mononuclear cells (PBMCs)

Peripheral blood was obtained from the local blood bank as standard buffy coat preparations from healthy donors. Heparin (2001.U./ml blood, Liquemine, Roche) was used to prevent clotting. Peripheral blood mononuclear cells (PBMCs) were isolated by centrifugation in LSM^{®} (1.077-1.080g/ml; ICN, Aurora, OH) at 800g (room temperature) for 30 min. PBMCs were collected from the interphase and washed twice in RPMI containing 20 mM Hepes (500g for 15 min, 300g for 5 min). In order to remove erythrocytes, PBMCs were treated with ALT buffer (140 mM ammonium chloride, 20 mM Tris, pH 7.2) for 3 min at 37°C. PBMCs were washed twice with RPMI containing 20 mM Hepes (200g for 5 min).

### Generation of dendritic cells from peripheral blood monocytes.

Monocytes were isolated from PBMCs by positive sorting using anti-CD14 magnetic beads (Miltenyi Biotech, Auburn, CA) according to the manufacturer's protocol. Monocytes were cultured in RPMI supplemented with 1% non-essential amino acids (Gibco, BRL, Rockville, MD), 50 ng/ml recombinant human granulocyte macrophage-colony stimulating factor (GM-CSF; S.A. 1.1x10⁷U/mg) (Leucomax; Novartis, Basel Switzerland) and3 ng/ml recombinant human IL-4 (S.A. 2.9x10⁴U/mg) (R&D Systems, Minneapolis, MN). Monocytes were seeded at 0.3 x 10⁶/ml in 6-well plates (Costar) for 5 days to obtain immature dendritic cells.

The quality of monocyte-derived immature dendritic cells was routinely monitored by flow-cytometric analysis conforming to the phenotype: CD1a (high), CD3 (neg.), CD 14 (low), CD19 (neg.), CD56 (neg.), CD80 (low), CD83 (neg.), CD86 (low) and HLA-DR (high). In contrast, mature dendritic cells (cf. below) display the following phenotype: CD1a (low), CD80 (high), CD83 (high), CD86 (high) and HLA-DR (high). Monoclonal antibodies against CD1a, CD3, CD14, CD19, CD56, CD80, CD83, CD86 as well as the respective isotype controls were purchased from Pharmingen (San Diego, CA).

### Exposure of dendritic cells to necrotic melanoma cells

Melanoma cells lines were rendered necrotic by 4 cycles of freezing in liquid nitrogen and subsequent thawing at room temperature. The percentage of necrotic cells was monitored by light microscopy. To feed dendritic cells with melanoma cell-derived antigen, 6 x 10⁶ immature dendritic cells were exposed to 1.8 x 10⁷ necrotic cells (3:1 ratio). At the same time maturation of dendritic cells was induced by adding 10 ng/ml recombinant human tumor necrosis factor (TNFalpha; S.A. 1.1x10⁵U/mg). As a control, 6 x 10⁶ dendritic cells were incubated with TNFalpha alone.

After 24-48 hrs of co-culture, mature dendritic cells were harvested by centrifugation at 300g for 10 min. Cells were washed with RPMI containing 10% FCS and transferred to an eppendorf tube. After centrifugation at 400g for 3 min, the supernatant was completely removed and the cells were frozen at -70°C.

### Generation of anti-HLA class II beads

The anti-HLA-DR monoclonal antibody (mAb) L243 (ATCC, Manassas, VA) was produced by culturing the respective mouse hybridoma cell line. mAb L243 was purified using ProteinA sepharose (Pharmacia, Uppsala, Sweden) and immobilized to CNBr-activated sepharose beads (Pharmacia) at a final concentration of 2.5 mg/ml, according to the manufacturer's protocol. L243 beads were stored in PBS containing 0.1% Zwittergent 3-12 (Calbiochem, La Jolla, CA).

### Nano-scale purification of HLA-DR-peptide complexes

Pellets of frozen dendritic cells were resuspended in 10-fold volume of ice cold lysis buffer (1% Triton-X-100, 20 mM Tris, pH 7.8, 5 mM MgCl₂, containing protease inhibitors chymostatin, pepstatin, PMSF and leupeptin (Roche, Mannheim, Germany)) and lysed in a horizontal shaker at 1000 rpm, 4°C for 1h. The cell lysate was cleared from cell debris and nuclei by centrifugation at 2000g, 4°C for 10 min. The lysate was co-incubated with L243 beads (5-10 µl L243 beads per 100 µl cell lysate) in a horizontal shaker at 1000 rpm, 4°C for 2 hrs. Immunoprecipitated HLA-DR-peptide complexes bound to L243 beads were sedimented by centrifugation at 2000g, 4°C for 5 min and washed three times with 300 µl 0.1% Zwittergent 3-12 (Calbiochem) in PBS.

The efficacy of depletion of HLA-DR-peptide complexes was monitored by analyzing the respective cell lysates before and after immunoprecipitation. In parallel, aliquots of the beads were analyzed by western blotting using the anti-HLA-DRa-specific mAb 1B5 (Adams, T.E. et al., Immunology 50 (1983) 613-624).

### Elution of HLA-DR-associated peptides

HLA-DR-peptide complexes bound to L243 beads were resuspended in 400 µl H₂O (HPLC-grade; Merck, Darmstadt, Germany), transferred to an ultrafiltration tube, Ultrafree MC, 30 kD cut-off (Millipore, Bedford, MA) and washed 10 times with 400 µl H₂O (HPLC-grade) by centrifugation for 2-4 min at 14000 rpm at 4°C. For eluting the bound peptides, 50 µl 0.1% trifluoracetic acid (Fluka, Buchs, Switzerland) in H₂O (HPLC-grade) was added and incubation was performed for 30 min at 37°C. Eluted peptides were collected in a new eppendorf tube by centrigugation of the Ultrafree unit at 14000 rpm for 3 min at RT and immediately lyophilized in a Speed-Vac™ vacuum centrifuge.

### Fractionation of peptides by nano-HPLC

Lyophilized peptides eluted from HLA-DR molecules were resolved in 0.05% trifluoroacetic acid, 5% acetonitrile (Merck, Darmstadt, Germany) in H₂O, (HPLC-grade) and separated on a 75 µm x 15 cm C18 PepMap capillary (C18; 3µm; 100 Å) (LC-Packings, Amsterdam, Netherlands) connected to a FAMOS™ autosampler and an ULTIMATE™ nano-flow HPLC (Dionex, Olten, Switzerland). The following non-linear gradient at a constant flow rate of 200 nl/min was used: 0-40min 5-50% system B; 40-50 min 50-90% system B. System A was 0.05% trifluoroacetic, 5% acetonitrile/H₂O and system B was 0.04% trifluoroacetic, 80% acetonitrile/H₂O. The separation was monitored via dual UV absorption at 214 nm and 280 nm. Fractions (400 nl) were collected using the fraction collector PROBOT™ (BAI, Weiterstadt, Germany) and spotted onto an AnchorChip 600/384 MALDI-MS target (Bruker, Bremen, Germany).

### Sequence analysis of peptides by mass spectrometry

### MALDI-TOF mass spectrometry

Peptides spotted onto an AnchorChip plate were co-cristallized with matrix (10 mg/ml; a-cyano-4-hydroxy-cinnamic acid (Merck, Darmstadt, Germany), 50% acetonitrile, 0.1% trifluoroacetic acid). For qualitative analysis of the whole peptide repertoire, samples were analyzed on an Ultraflex™ MALDI-TOF mass spectrometer (Bruker, Bremen, Germany), according to the manufacturer's protocol.

### Ion Trap MS/MS mass spectrometry

To perform high-throughput sequencing of complex peptide mixtures, the MudPIT (multidimensional protein identification technology) was used (Washburn MP et al., Nat Biotechnol 19 (2001), 242-247) which is based on a liquid chromatographic fractionation followed by mass spectrometric sequencing.

To this end, the lyophilized peptides eluted from HLA molecules were resuspended in a buffer containing 5% (v/v) acetonitrile, 0.5% (v/v) acetic acid, 0.012% (v/v) heptafluoro butyric acid (HFBA) and 5% (v/v) formic acid. The sample was separated on a fused-silica microcapillary column (100 µm i.d. × 365 µm) generated by a Model P-2000 laser puller (Sutter Instrument Co., Novato, CA). The microcolumn was packed with 3 µm / C18 reverse-phase material (C18-ACE 3 µm [ProntoSIL 120-3-C18 ACE-EPS, Leonberg, Germany]) followed by 3 cm of 5 µm cation exchange material (Partisphere SCX;Whatman, Clifton, NJ).

A fully automated 8-step gradient separation on an Agilent 1100 series HPLC (Agilent Technologies, Waldbronn, Germany) was carried out, using the following buffers: 5% ACN/0.02% HFBA/0.5% acetic acid (buffer A), 80% ACN/0.02% HFBA/0.5% acetic acid (buffer B), 250 mM ammonium acetate/5% ACN/0.02% HFBA/0.5% acetic acid (buffer C), and 1.5 M ammonium acetate/5% ACN/0.02% HFBA/0.5% acetic acid (buffer D). The first step of 106 min consisted of a 100 min gradient from 0 to 80% buffer B and a 6 min hold at 80% buffer B. The next 6 steps (106 min each) are characterized by the following profile: 5 min of 100% buffer A, 2 min of x% buffer C, 5 min of 100% buffer A, a 3 min gradient from 0 to 10% buffer B, a 55 min gradient from 10 to 35% buffer B, a 20 min gradient from 35 to 50% buffer B, a 16 min gradient from 50 to 80% buffer B. The 2 min buffer C percentages (x) in steps 2-7 were as follows: 10, 20, 30, 40, 70, 90, and 100%. Step 8 consisted of the following profile: a 5 min 100% buffer A wash, a 20 min salt wash with 100% buffer D and a 100 min gradient from 0-80% buffer B.

The HPLC column was directly coupled to a Finnigan LCQ ion trap mass spectrometer (Finnigan, Bremen, Germany) equipped with a nano-LC electrospray ionization source. Mass spectrometry in the MS-MS mode was performed according to the manufacturer's protocol. The identification of peptides was done by the sequest algorithm against the swiss.fasta database.

### MALDI-PSD mass spectrometry

As an alternative to doing sequence analysis by ion trap MS/MS, as described above, MALDI-PSD analysis was performed on a Bruker Ultraflex TOF/TOF mass spectrometer (Bruker, Bremen, Germany) using the software FLEXControl 1.1 Alpha for data acquisition. Calibration was achieved by using a tryptic digest of human serum albumin (Merck, Darmstadt, Germany). Peptide mixtures were first scanned in a reflectron mode. Peptides of interest were then selected for lift mode (MALDI-PSD analysis). The peptide fragmentation spectra obtained were automatically evaluated using the Xmas 5.1.2 and Biotools 2.1 Software (Bruker) and used for sequence identification in a non-redundant protein database using the MASCOT algorithm (http://www.matrixscience.com).

### Example 1

Strategy 1 (Fig. 1A) was used to identify peptides associated to HLA-DR molecules expressed on the surface of peripheral blood mononuclear cells (PBMCs). PBMCs were isolated from peripheral blood by Ficoll density gradient centrifugation. From 50 ml blood 5.3 x10⁷ PBMCs were recovered. The cell types typically present in PBMCs are T lymphocytes (about 50%), B lymphocytes (5-10%), monocytes (15-25%) and natural killer cells (about 6%). Peripheral blood dendritic cells are also present but only in very low amounts (< 0.5%). Analysis of PBMCs by flow cytometry revealed that both B cells and monocytes express considerable amounts of HLA-DR molecules, while natural killer cells and T cells stain negative. The small amount of dendritic cells in PBMCs cannot be visualized by FACS. Human T cells are able to up-regulate HLA-DR upon activation, however, activated T cells are normally absent from peripheral blood.

Although the number of B cells present in PBMCs is 2 to 3-fold lower compared to monocytes, their HLA-DR expression level is about 2-fold higher. This means that in lysates from PBMCs the number of HLA-DR molecules originating from B cells is comparable to the number of HLA-DR molecules from monocytes.

PBMCs were lysed in TX-100 and HLA-DR molecules were precipitated using anti-DR mAb L243. Precipitation was controlled by western blot analysis using anti-DRalpha mAb 1B5. Quantitative western blot analysis using purified HLA-DR molecules as a reference revealed that about 200 ng HLA-DR was purified from 5.3 x 10⁷ cells. HLA-DR associated peptides were eluted in 0.1% TFA and the peptide mixture was fractionated using 2-dimensional cation-exchange/ reversed phase liquid chromatography (MudPit). Sequencing was done by high-throughput ion trap mass spectrometry and data base search was performed using human data bank "humangp". The peptides identified with a cross-correlation > 2.0 are listed in Table 1.

27 peptides could be identified: 8 peptides were derived from human serum albumin and constituted a nested set of peptides typical for MHC class II associated peptides (N-and C-terminal elongation / truncation variants of the same epitope); 3 peptides were derived from apolipoprotein AII, again representing only one epitope; 3 peptides were derived from alpha1 anti-trypsin and represented one epitope; 4 peptides from protein disulfide isomerase related protein ERp72 (1 epitope). The last 9 peptides were derived from different proteins and, thus, represented 9 different epitopes.

15 peptides (4 epitopes) were derived from extracellular proteins that are major constituents of human serum: serum albumin is the most abundant protein in serum, apolipoprotein All is a constituent of high density lipoproteins (HDLs), alpha 1 anti-trypsin is a well known serum protease inhibitor. Ferritin light chain (donor of peptide nr. 10) is present in virtually all cells and at low concentration in plasma. Most likely, these proteins were internalized by fluid phase uptake, and after proteolytic processing the respective peptides were loaded onto HLA-DR molecules inside the APC (monocyte or B cell). Alternatively, these proteins or fragments of the respective proteins could have bound to cell surface HLA-DR molecules.

The lysosomal-associated multi-transmembrane protein (lam5) is the donor of peptide nr. 4 and is expressed in haematopoietic cells. The subcellular localization of lam 5 is the lysosome, so it is already present in the loading compartment of HLA-DR molecules, where it can bind before or after proteolytic cleavage. HLA class I molecules (giving rise to peptide nr. 19) are present in nearly all nucleated cells, thus it is likely to be derived from the APC itself. Alternatively, HLA class I can be taken up from the serum where shedded HLA class I molecules have been described.

PDI ERp72 is an ER resident protein that has been described to be expressed in muscle and lung but also in a lymphoblastoid cell line. ERp72 gave rise to peptides nr. 20 to 23. Pyruvate kinase is a cytosolic protein and exists in several isoforms with isoform M1 being expressed in muscle, heart and brain, and isoform M2 is described for fetal tissue. The epitope (peptides 20-23) is present in both isoforms. Peptide nr. 24 is derived from actin alpha 1, a cytosolic protein that is highly expressed in skeletal muscle. Thus, all three epitopes could be derived from muscle cells that might have released their cellular proteins into the serum due to micro-tissue injury or damage.

Peptide nr. 5 is derived from F-box helicase 1, however, little is known, as yet, about the tissue expression of this protein.

Peptides nr. 25, 26, 27 are derived from proteins that could be allocated to chromosomes 17, 6 and 4, respectively, but there was no information about the respective proteins or their function, tissue expression or subcellular localization.

The bovine analogue (TPTLVEVSRSLGKVGTR) of the serum albumin epitope (peptides nr. 11-18) has been described as a HLA-DR-associated epitope in context of the DR alleles DRB1*1101/ / DRB1*1104 (Verreck FA et al., Immunogenetics 1996; 43, 392-397). It has been identified by Edman sequencing of self-peptides derived from cultured EBV-transformed B cells. The peptide binding motif of both of these alleles requires an aromatic or aliphatic residue at position P1, an aliphatic residue at P4 and a basic residue at P6 (Verreck FA et al., Immunogenetics 1996; 43, 392-397). These requirements are also fulfilled in the human serum albumin epitope derived from PBMCs and identified here (P1 = L, P4 = V, P6 = R), (peptides 11-18), but also in peptides 1-4, 6-10, 20-23 and 27 (Table 1). Moreover, an epitope overlapping with the alpha1 anti-trypsin peptide found here has been described in EBV B cells in the context of DRB1*0402 (Friede T et al., 1996, Biochim Biophys Acta 1996; 1316; 85-10).

The DR allele coexpressed with DRB1*1101 is DRB3*0202, which has the following requirements for peptide binding: aromatic or aliphatic residue at position P1, an asparagine at P4 and a polar residue at P6 (Verreck FA et al., Immunogenetics 1996; 43, 392-397). These requirements are fulfilled by peptides 5 and 19. Thus, most of the identified peptides (n=21) contain the same motif, indicating that they are derived from the same HLA-DR allotype or from more than on HLA-DR allotype sharing a common motif.

### Example 2

Strategy 2 (Fig. 1B) was used to identify novel peptides associated to HLA-DR molecules expressed on the surface of dendritic cells that are exposed to a potential antigen. In this case, the antigenic source was a commercially available vaccine against influenza virus, denoted as INFLEXAL Berna V (Berna, Bern, Switzerland).

Dendritic cells were differentiated from peripheral blood monocytes and cultured in a concentration of 0.5 x10⁶ cells/ml. 6 x10⁶ dendritic cells were exposed to the vaccine INFLEXAL Berna V (Berna, Bern, Switzerland) for 24 hrs by adding INFLEXAL Berna V at a concentration of 100 µl/ml (corresponding to 3 µg/ml hemagglutinin derived from influenza virus). At the same time maturation of dendritic cells was induced by adding TNFalpha (10 ng/ml). As a control, the same amount of dendritic cells (6 x10⁶) was cultured in the absence of the antigen, but in then presence of TNFalpha (10 ng/ml).

Both sets of dendritic cells were lysed in detergent TX-100 and HLA-DR molecules were precipitated by using anti-DR mAb L243 immobilized to sepharose beads. HLA-DR associated peptides were eluted with 0.1% TFA and analyzed by MALDI-MS (Fig. 2A): The upper panel shows the complex mixture of HLA-DR-associated self-peptides from unpulsed DCs. The 3 dominant peptides displaying a mass-to-charge ratio of m/z = 2334, m/z = 2545 and m/z = 2676, correspond to variants of CLIP, the class II associated invariant chain peptide (Riberdy JM et al., Nature 1992; 360, 474-477), a dominant self peptide in mature dendritic cells. The lower panel shows the peptide repertoire of DCs that were pulsed with INFLEXAL. Comparison of both MS spectra revealed that 3 novel signals became dominant in the MALDI-MS peptide profile of DCs upon contact with the INFLEXAL vaccine. These 3 novel signals appeared at m/z = 1969.4, m/z = 2097.6 and m/z = 2196.6 (Fig. 2A).

The hemagglutinin proteins contained in INFLEXAL Berna V are derived from 3 different influenza strains: strain A / New Caledonia / 20 / 99; strain A / Panama / 2007 / 99 ; strain B / Yamanashi / 166/98 (according to the recommendation of the WHO taking into account the genetic diversity among the circulating viruses (Lindstrom SE et al., J. Virol. 1999, 73, 4413-4426)).

A search for the identified masses in the three different hemagglutinin sequences of the above mentioned influenza strains revealed that all three peptides represented length variants of one epitope (SEQ ID NOs: 86, 87, 88, 89) located in influenza hemagglutinin, strain B / Yamanashi / 166/98 (Fig.2B; SEQ ID NO: 90).

This epitope HA(253-271) KPGKTKTIVYQRGILLPQK (SEQ ID NO: 88) contains the MHC peptide binding motif for DRB1*0101 and DRB5*0101 using I-260 as P1, Q-264 as P4 and L269 as P9 anchor residue (anchor residues are underlined).

MHC peptide binding studies using the synthetic peptide with the amino acid sequence KPGKTKTIVYQRGILLPQ confirmed the binding capacity to alleles DRB1*0101 and DRB5*0101 and revealed the same for DRB1*0401. Thus, the newly identified epitope derived from influenza hemagglutinin (strain B / Yamanashi / 166 / 98) reveals an epitope with promiscuous binding capacity.

### Example 3

Furthermore, strategy 2 (Fig. 1B) was used to identify novel HLA-DR-associated tumor peptides. Thus, dendritic cells were exposed to a necrotic melanoma cell line, UKRV-Mel-15a.

3 x 10⁶ cells dendritic cells were co-incubated with 9 x 10⁶ necrotic cells of the melanoma line UKRV-Mel-15a and cultured for 24 hrs in presence of TNFalpha (10 ng/ml). As a control, 3 x 10⁶ cells dendritic cells were cultured in presence TNFalpha (10 ng/ml) only.

Both sets of dendritic cells were lysed in detergent TX-100 and HLA-DR molecules were precipitated using anti-DR mAb L243. HLA-DR associated peptides were eluted with 0.1%. TFA and analyzed by MALDI MS (Fig. 3A):

In this example, the HLA-DR associated peptides from both DC cultures were compared by MALDI-MS spectrometry and only the peptide signals contained in the profile of DCs pulsed with melanoma cells were used to identify new epitopes by successive sequencing.

MALDI-MS analysis revealed one dominant signal with an observed mass of m/z = 1820.6 in the spectrum of pulsed DCs as compared to unpulsed DCs (Fig. 3A).

Sequencing by MALDI-PSD fragmentation resulted in a novel epitope derived from the tumor antigen vimentin: vimentin(202-217) with the amino acid sequence TLQSFRQDVDNASLA (Fig. 3B). Sequence analysis by ion trap MS-MS confirmed this sequence (Fig. 3C).

Vimentin(202-217) and several other known melanoma antigens share a common motif suitable for binding to HLA-DR4 B1*0401 molecules (Table 2). In contrast to the typical DRB1*0401 peptide binding motif derived from self- and foreign antigenic peptides, the peptides derived from melanA, CDC27, tyrosinase and vimentin display asparartic acid (D) instead of threonine (T) or serine (S) at anchor position P6. The relevance of this peculiarity remains to be investigated.

Vimentin is known to be a marker protein in a variety of benign and malign tumors. Together with melanA/MART-1, tyrosinase and S100, vimentin is routinely used to trace melanoma cells in clinical specimens from melanoma patients. Interestingly, melanoma clones with low invasive potential have a high vimentin expression, whereas vimentin is downregulated in highly invasive melanoma cell clones (Gutgemann A et al., Arch Dermatol Research 2001; 293, 283-290). In contrast, enhanced expression of vimentin is observed in poorly differentiated and metastatic prostate carcinoma (Lang SH et al., Prostate 2002; 52, 253-263). Moreover, vimentin is overexpressed in human renal cell carcinoma in relation to normal renal tissue (Stassar MJ et al. Br. J. Cancer 2001; 85, 1372-1382). Likewise, >95% of tumor cells in classical Hodgkin's lymphoma are vimentin positive, whereas T-cell-rich B-cell lymphomas are negative for vimentin (Rudiger T et al., Am J Surg Path 1998, 22, 1184-91).

Vimentin(202-217) peptide identified by the method of the invention is the first vimentin derived HLA class II restricted epitope described so far.

### Example 4

In this example strategy 2 (Fig. 1B) was used to identify as many peptides as possible which were bound to HLA-DR molecules of dendritic cells (DCs) after TNFalpha-induced maturation and exposure to potential antigens. Sequencing was done by high-throughput ion trap MS/MS technology.

Thus, 5 x 10⁶ cells dendritic cells were co-incubated with 1.5 x 10⁷ necrotic cells of the melanoma line UKRV-Mel-20c and cultured for 24 hrs in presence of TNFalpha (10 ng/ml). As a control, 5 x 10⁶ cells dendritic cells were cultured in presence TNFalpha (10 ng/ml) only.

Both sets of dendritic cells were lysed in detergent TX-100 and HLA-DR molecules were precipitated using anti-DR mAb L243. HLA-DR associated peptides were eluted with 0.1% TFA and analyzed by LC-high-throughput ion trap MS/MS technology.

The peptide sequences identified from unpulsed DCs (control) are given in Table 3, whereas the peptide sequences identified from DCs pulsed with necrotic melanoma cells are listed in Table 4.

35 individual peptide sequences from HLA-DR molecules of DCs were identified in the absence of melanoma cells, and 40 peptide sequences were found in the presence of UKRV-Me120c melanoma cells. Comparison of the peptide sequences revealed that 21 peptides are identical (nr. 1-21), 14 sequences (11 epitopes) are specific for unpulsed DCs and 17 sequences (9 epitopes) are only presented after melanoma cell pulse.

7 of the 9 peptides induced by melanoma cells share the binding motif of DRB1*0405 (Table 5). Importantly, 3 of the 9 melanoma cell induced epitopes are derived from known tumor marker proteins, namely translation factor IF-4A1, translation factor EF-1alpha and interferon-gamma (IFNgamma)-inducible P78.

The translation initiation factor IF-4A1 is consistently overexpressed in melanoma cell lines in relation to normal human melanocytes. IF-4A1 overexpression seems to be an important feature of melanoma cells and might contribute to their malignant transformation (Eberle J et al., Int. J. Cancer 1997; 71, 396-401).

A wide evidence suggests the involvement of ribosomal elongation factors (EF) at the onset of oncogenesis. Altered expression of EF-1alpha, a core component of protein synthesis, has been linked to transformed phenotypes in several studies. Overexpression of EF-1alpha mRNA has been correlated with increased metastatic potential in mammary adenocarcinoma and EF-1alpha has a considerable degree of homology with the prostate oncogene PTI-1 (Gopalkrishnan RV et al., Int J.Biochem Cell Biol 1999; 31, 151-162; Edmonds BT et al., J Cell Sci 1996; 109, 2705-2714).

A naturally processed self-peptide derived from EF-1alpha containing the same epitope as peptides nr. 42 and 43 (Table 4) has also been eluted from HLA-DR molecules purified from EBV transformed B cell lines (Verreck FA et al., Immunogenetics 1996; 43, 392-397).

Prostate cancer progression from a hormon-dependent to a hormon-independent state includes a cascade of genetic alterations caused by activation of oncogenes and/ or inactivation of tumor suppressor genes. Several genes were identified which are highly overexpressed in androgen-independent cancer cell lines. Amongst others, the interferon-inducible genes 1-8U and p78 were identified (Markku H et al., Lab Invest. 2000; 80, 1259-1268.).

Thus, the peptides derived from translation factor IF-4A1, translation factor EF-1alpha and IFNgamma-inducible P78 identified by the method of the invention are new candidate tumor antigens that can be used as diagnostic markers or therapeutic vaccines.

**TABLE 1**

| **HLA-DR bound peptides derived from peripheral blood mononuclear cells** | | | | | |
|---|---|---|---|---|---|
| SEQ ID NO.: | PEP. Nr. | LENGTH | OBSERVED MASS | SEQUENCE^{a} | PROTEIN SOURCE |
| 1 | 1 | 16 | 1756.4 | SKEQLTPLIKKAGTEL | apolipoprotein All |
| 2 | 2 | 15 | 1643.6 | SKEQLTPLIKKAGTE | apolipoprotein All |
| 3 | 3 | 14 | 1556.8 | KEQLTPLIKKAGTE | apolipoprotein All |
| 4 | 4 | 16 | 1765.6 | SVLLFIEHSVEVAHGK | lysosomal-associated multi-transmembrane protein lam5 |
| 5 | 5 | 17 | 1795.8 | VDGILSNCGIEKESDLC | F-box DNA helicase 1 |
| 6 | 6 | 17 | 1888.2 | GTQGKIVDLVKELDRDT | alpha1 anti trypsin |
| 7 | 7 | 16 | 1830.8 | TQGKIVDLVKELDRDT | alpha1 anti-trypsin |
| 8 | 8 | 14 | 1614.5 | TQGKIVDLVKELDR | alpha1 anti-trypsin |
| 9 | 9 | 14 | 1623.6 | GKNIKIISKIENHE | pyruvate kinase M1/M2 |
| 10 | 10 | 14 | 1655.5 | LDEEVKLIKKMGDH | ferritin light chain |
| 11 | 11 | 22 | 2353.9 | TPTLVEVSRNLGKVGSK CCKPH | serum albumin |
| 12 | 12 | 18 | 1872.5 | STPTLVEVSRNLGKVGS K | serum albumin |
| 13 | 13 | 17 | 1785.6 | TPTLVEVSRNLGKVGSK | serum albumin |
| 14 | 14 | 17 | 1756.8 | VSTPTLVEVSRNLGKVG | serum albumin |
| 15 | 15 | 17 | 1744.3 | STPTLVEVSRNLGKVGS | serum albumin |
| 16 | 16 | 16 | 1657.4 | STPTLVEVSRNLGKVG | serum albumin |
| 17 | 17 | 15 | 1569.6 | TPTLVEVSRNLGKVG | serum albumin |
| 18 | 18 | 14 | 1513.4 | TPTLVEVSRNLGKV | serum albumin |
| 19 | 19 | 16 | 1852.5 | GKDYIALNEDLRSWTA | HLA class I heavy chain |
| 20 | 20 | 16 | 1794.9 | GYPTIKILKKGOAVDY | PDI ERp72 |
| 21 | 21 | 15 | 1737.9 | YPTIKILKKGOAVDY | PDI ERp72 |
| 22 | 22 | 15 | 1631.1 | GYPTIKILKKGOAVD | PDI ERp72 |
| 23 | 23 | 14 | 1574.8 | YPTIKILKKGOAVD | PDI ERp72 |
| 24 | 24 | 16 | 1791.1 | SYELPDGOVITIGNER | actin alpha 1 |
| 25 | 25 | 14 | 1624.6 | SVILKILPSYOEPH | *(chr 17)* |
| 26 | 26 | 14 | 1466.8 | AKIHIDIVLVGGSTR | *(chr 6)* |
| 27 | 27 | 15 | 1716.1 | NALLVRTKKVPOVS | *(chr 4)* |

| | | | | | |
|---|---|---|---|---|---|
| ^{a}the 9-mer core region containing the peptide binding motif of each peptide is underlined | | | | | |

**TABLE 2**

| **HLA-DR4 (B1*0401)-associated melanoma antigens** | | | | | | |
|---|---|---|---|---|---|---|
| SEQ ID NO: | PEP. Nr. | LENGTH | SEQUENCE^{a} | POSTITION | PROTEIN SOURCE | REF. |
| 28 | 54 | 15 | RNGYRALM**D**KSLHVG | 51-65 | Melan-A | ^{b} |
| 29 | 55 | 15 | MNFSWAMDL**D**FKGAN | 768-772 | CDC27 | ^{b} |
| 30 | 56 | 13 | SYLQDS**D**PDSFQD | 448-462 | Tyrosinase | ^{b} |
| 31 | 53 | 16 | TLQSFRQDV**D**NASLAR | 202-217 | Vimentin | this study |

| | | | | | | |
|---|---|---|---|---|---|---|
| ^{a} The sequences of the peptides are aligned according to the peptide binding motif of HLA-DR4 (DRB1 *0401): P1 anchor: W,Y,F; P4 anchor: D,E,L. The peculiarity of "D" (instead of T,S or N) at anchor position 6 is marked in bold. ^{b}R.-F. Wang, Trends in Immunology 22, 269-276 (2001) | | | | | | |

**TABLE 3**

| **HLA-DR bound peptides derived from mature dendritic cells** | | | | | | |
|---|---|---|---|---|---|---|
| SEQ ID NO: | PEP. Nr. | LENGTH | OBSERVED MASS | SEQUENCE ^{a} | PROTEIN SOURCE | CELLULAR COMPARTMENT |
| 32 | 1 | 17 | 1829.0 | FPEPIKLDNKNDRAKAS | Rab-7 | cytosol |
| 33 | 2 | 16 | 1746.9 | HTGALYRIGDLQAFQG | CD98 | surface |
| 34 | 3 | 14 | 1552.8 | TGALYRIGDLQAFQ | CD98 | surface |
| 35 | 4 | 16 | 1720.0 | AKNQVAMNPTNTVFDA | HSC 70 | cytosol |
| 36 | 5 | 17 | 1786.0 | NVLRIINEPTAAAIAYG | HSP 70 | cytosol |
| 37 | 6 | 18 | 1899.1 | LNVLRIINEPTAAAIAYG | HSP 70 | cytosol |
| 38 | 7 | 14 | 1586.9 | IDKVISTITNNIQQ | TGF-induc. Ig | surface |
| 39 | 8 | 14 | 1667.8 | DDVILNEPSADAPA | Integr. MP2B | surface |
| 40 | 9 | 15 | 1632.9 | NSNQIKILGNQGSFL | CD4 | surface |
| 41 | 10 | 16 | 1642.0 | NKEGLELLKTAIGKAG | α-Enolase | cytosol |
| 42 | 11 | 15 | 1769.9 | KVVVYLQKLDTAYDD | Cathepsin C | endosome |
| 43 | 12 | 16 | 1883.0 | KKVVVYLQKLDTAYDD | Cathepsin C | endosome |
| 44 | 13 | 16 | 1898.0 | KVVVYLQKLDTAYDDL | Cathepsin C | endosome |
| 45 | 14 | 15 | 1746.9 | YPRISVNNVLPVFDN | Cathepsin D | endosome |
| 46 | 15 | 16 | 1800.9 | TTAFQYIIDNKGIDSD | Cathepsin S | endosome |
| 47 | 16 | 17 | 1871.9 | TTAFQYIIDNKGIDSDA | Cathepsin S | endosome |
| 48 | 17 | 16 | 1812.9 | LPGQLKPFETLLSQNQ | GSH-S-Transf. | cytosol |
| 49 | 18 | 17 | 1870.0 | LPGQLKPFETLLSQNQG | GSH-S-Transf. | cytosol |
| 50 | 19 | 16 | 1830.9 | VSNEIVRFPTDQITPD | Myeloper oxid | endosome |
| 51 | 20 | 17 | 1886.0 | VDEVTIVNILTNRSNAQ | Annexin-II | cytosol |
| 52 | 21 | 17 | 1897.4 | TDGKDYIALNEDLSSWT | HLA-B | surface |
| 53 | 22 | 14 | 1600.9 | LAVVKSIRSIPYLA | Annexin-V | cytosol |
| 54 | 23 | 15 | 1714.1 | LLAVVKSIRSIPYLA | Annexin-V | cytosol |
| 55 | 24 | 13 | 1614.9 | VADKIQLINMLDK | PG-Kinase | cytosol |
| 56 | 25 | 14 | 1717.8 | DQVIKVFNDMKVRK | Cofilin | cytosol |
| 57 | 26 | 15 | 1680.9 | LRTIDVFDGNSGKMM | GAP-DH | cytosol |
| 58 | 27 | 15 | 1628.9 | GKVDIVAINDPFIDL | GAP-DH | cytosol |
| 59 | 28 | 14 | 1576.8 | DDIRGIQSLYGDPK | Metallo-Elastase | extracell. |
| 60 | 29 | 15 | 1647.8 | ADDIRGIQSLYGDPK | Metallo-Elastase | extracell. |
| 61 | 30 | 15 | 1600.9 | SSNVVHLIKNAYNKL | Integrin-β | surface |
| 62 | 31 | 16 | 1756.9 | LNQELRADGTVNQIEG | Apolipoprotein D | extracell. |
| 63 | 32 | 15 | 1611.8 | GPLKFLHQDIDSGQG | Man-6-P-Rez. | surface |
| 64 | 33 | 17 | 1880.9 | GPLKFLHQDIDSGQGIR | Metallo-Elastase | cytosol |
| 65 | 34 | 17 | 1906.9 | VKSEIIPMFSNLASDEQ | Prot.-Phosphat. 2A | cytosol |
| 66 | 35 | 16 | 1821.8 | GSHSMRYFHTAMSRPG | HLA-B | surface |

| | | | | | | |
|---|---|---|---|---|---|---|
| ^{a}the P1 anchor of the HLA-DR bound peptide is underlined | | | | | | |

**TABLE 4**

| **HLA-DR bound peptides derived from mature dendritic cells pulsed with the melanoma cell line UKRV-Mel-20c** | | | | | | |
|---|---|---|---|---|---|---|
| SEQ ID NO: | PEP. Nr. | LENGTH | OBSERVED MASS | SEQUENCE^{a} | PROTEIN SOURCE | CELLULAR COMPARTMENT |
| 32 | 1 | 17 | 1829.0 | FPEPIKLDNKNDRAKAS | Rab-7 | cytosol |
| 33 | 2 | 16 | 1746.9 | HTGALYRIGDLQAFQG | CD98 | surface |
| 34 | 3 | 14 | 1552.8 | TGALYRIGDLQAFQ | CD98 | surface |
| 35 | 4 | 16 | 1720.0 | AKNQVAMNPTNTVFDA | HSC 70 | cytosol |
| 36 | 5 | 17 | 1786.0 | NVLRIINEPTAAAIAYG | HSP 70 | cytosol |
| 37 | 6 | 18 | 1899.1 | LNVLRIINEPTAAAIAYG | HSP 70 | cytosol |
| 38 | 7 | 14 | 1586.9 | IDKVISTITNNIQQ | TGF-induce. Ig | membrane |
| 39 | 8 | 16 | 1667.8 | DDVILNEPSADAPA | Integr. MP2B | membrane |
| 40 | 9 | 15 | 1632.9 | NSNQIKILGNQGSFL | CD4 | surface |
| 41 | 10 | 16 | 1642.0 | NKEGLELLKTAIGKAG | α-Enolase | cytosol |
| 42 | 11 | 15 | 1769.9 | KVVYLQKLDTAYDD | Catheps C | endosome |
| 43 | 12 | 16 | 1883.0 | KKVVVYLQKLDTAYDD | Catheps in C | endosome |
| 44 | 13 | 16 | 1898.0 | KWVYLQKLDTAYDDL | Catheps in C | endosome |
| 45 | 14 | 16 | 1746.9 | YPRISVNNVLPVFDN | Catheps in D | endosome |
| 46 | 15 | 16 | 1800.9 | TTAFQYIIDNKGIDSD | Catheps in S | endosome |
| 47 | 16 | 17 | 1871.9 | TTAFQYIIDNKGIDSDA | Catheps in S | endosome |
| 48 | 17 | 16 | 1812.9 | LPGQLKPFETLLSQNQ | GSH-S-Transf. | cytosol |
| 49 | 18 | 17 | 1870.0 | LPGQLKPFETLLSQNQG | GSH-S-Transf. | cytosol |
| 50 | 19 | 16 | 1830.9 | VSNEIVRFPTDQITPD | Myelope roxid. | endosome |
| 51 | 20 | 17 | 1886.0 | VDEVTIVNILTNRSNAQ | Annexin -II | cytosol |
| 67 | 21 | 17 | 1897.4 | DGKDYIALNEDLSSWTA | HLA-B | surface |
| 68 | 36 | 16 | 1879.1 | KRKTVTAMDVVYALKR | Histon H4 | nucleus |
| 69 | 37 | 16 | 1723.0 | KRKTVTAMDVVYALK | Histon H4 | nucleus |
| 70 | 38 | 14 | 1950.1 | AKRKTVTAMDVVYALKR | Histon H4 | nucleus |
| 71 | 39 | 14 | 1784.9 | SPKYIKMFVLDEADE | Transl. IF-4A1 | cytosol |
| 72 | 40 | 16 | 1915.9 | SPKYIKMFVLDEADEM | Transl. IF-4A1 | cytosol |
| 73 | 41 | 18 | 1886.1 | GSSRVLITTDLLARGIDV | Transl. IF-4A1 | cytosol |
| 74 | 42 | 16 | 1576.8 | TAQVIILNHPGQISAG | Transl. EF-1α | cytosol |
| 75 | 43 | 13 | 1647.8 | VIILNHPGQISAG | Transl. EF-1α | cytosol |
| 76 | 44 | 17 | 1600.9 | VYKVLKQVHPDTGISSK | Histon H2B | nucleus |
| 77 | 45 | 14 | 1756.9 | KVLKQVHPDTGISS | Histon H2B | nucleus |
| 78 | 46 | 15 | 1611.8 | KVLKQVHPDTGISSK | Histon H2B | nucleus |
| 79 | 47 | 14 | 1730.9 | KSKIEDIRAEQERE | IFN-γ induc. P78 | cytosol |
| 80 | 48 | 13 | 1601.9 | KSKIEDIRAEQER | IFN-γ induc. P78 | cytosol |
| 81 | 49 | 16 | 1761.9 | HNSLIASILDPYSNAF | Mannos e-Rec. | surface |
| 82 | 50 | 15 | 1831.9 | TTAYFLYQQQGRLDK | Inv. Chain | endosome |
| 83 | 51 | 17 | 1908.2 | NRQVNKKLNKTDLPKLL | K Channel | surface |
| 84 | 52 | 17 | 1930.0 | AEFLLHMLKNAESNAEL | Riboso mal L17 | cytosol |

| | | | | | | |
|---|---|---|---|---|---|---|
| ^{a}The P1 anchor of the HLA-DR bound peptide is underlined | | | | | | |

**TABLE 5**

| **HLA-DR bound peptides induced by melanoma cell line UKRV-Mel-20c and sharing the binding motif of DRB1*0405** | | | | | |
|---|---|---|---|---|---|
| SEQ ID NO: | PEP. Nr. | LENGTH | OBSERVED MASS | SEQUENCE^{a} | PROTEIN SOURCE |
| 85 | 39 | 15 | 1784.9 | SPKYIKMFVLDEADE | Transl. Factor elF-4A |
| 72 | 40 | 16 | 1915.9 | SPKYIKMFVLDEADEM | Transl. Factor elF-4A |
| 79 | 47 | 14 | 1730.9 | KSKIEDIRAEQERE | IFN-induc. P78 |
| 80 | 48 | 13 | 1601.9 | KSKIEDIRAEQER | IFN-induc. P78 |
| 82 | 50 | 15 | 1831.9 | TTAYFLYQQQGRLDK | Invariant Chain |
| 83 | 51 | 17 | 1908.2 | NRQVNKKLNKTDLPKLL | K - Channel |
| 84 | 52 | 17 | 1930.0 | AEFLLUMLKMAESNAEL | Ribos:Prot: L17 |

| | | | | | |
|---|---|---|---|---|---|
| ^{a} The sequences of the peptides are aligned according to the peptide binding motif of HLA-DR4 (DRB1*0405): P1 anchor: Y,F,I,L,V; P4 anchor: M,I,V; P9 anchor: E,D | | | | | |

## Claims

1. A method for isolating antigenic peptides in femtomolar amounts, which method consists of
(a) providing complexes of MHC II molecules with antigenic peptides isolated from cells, tissue samples or body fluid of a mammalian organism in an amount of 0.2 to 3 µg and (b) sequestering the complexes by immunoprecipitation, wherein the complexes are coupled by MHCII specific antibodies to beads,
c) washing the bound complexes of MHCII molecules with antigenic peptides with water or low salt buffer, wherein the low salt buffer is a Tris, phosphate or acetate buffer in a concentration range of 0.5 to 10mM and
d) eluting the associated antigenic peptides from the MHC II molecules.

2. The method according to claim 1, wherein the cells isolated from a mammalian organism are dendritic cells.

3. The method according to claims 1 or 2, wherein the sequestered complexes of MHC II molecules with antigenic peptides are washed with water in an ultrafiltration tube before eluting the peptides.

4. The method according to claims 1 to 3, wherein the antigenic peptides are eluted from the MHC II molecules using diluted acid.

5. The method according to any one of claims 1 to 3 wherein the antigenic peptides are eluted from the MHC II molecules at 37°C with diluted trifluoro acetic acid.

6. The method according to claims 1 to 5, wherein the isolated antigenic peptides are fractionated, sequenced and identified.

7. The method according to claim 6, wherein the isolated antigenic peptides are fractionated, sequenced and identified by methods comprising liquid chromatography and mass spectrometry.

8. The method according to claims 1 to 7, wherein the antigenic peptides are naturally-processed antigenic peptides or non-naturally processed antigenic peptides administered to the organism.

9. The method according to claims 1 to 8, wherein the cells are cells expressing MHC II molecules.

10. The method according to claims 1 to 9 wherein the mammalian organism is a human organism.

11. The method according to any of the claims 2 to 11
wherein the cells of step a) are MHC II expressing cells in a number providing 0.2 to 3 µg MHC II molecules and wherein the MHC II expressing cells are contacted with a source of potential antigen prior to the isolation of the MHC II molecule-antigenic peptide complexes.

12. The method according to claim 11, wherein the MHC II expressing cells are dendritic cells and wherein the dendritic cells are exposed to a potential source of antigen as immature dendritic cells at the same time as they are induced to mature to dendritic cells.

13. The method according to claims 11 to 12, wherein the source of potential antigen belongs to the group comprising tumor cells, tumor cell lines, pathogens, viral, bacterial and parasitic antigens, autoantigens, body fluids e.g. serum, synovial fluid, ascites.

14. The method according to claims 11 to 13, wherein the isolated antigenic peptides are fractionated, sequenced and identified and the antigenic peptides derived from the source of potential antigen are identified by comparing the peptides identified from cells which have been contacted with a source of potential antigen with those which have been identified from cells which have not been contacted with a source of potential antigen.

15. Use of the methods of claims 11 to 14 for the quality control of vaccines.

16. Use of the methods of claims 1 to 14 for immune monitoring of diseases.

17. Use of the methods of claims 1 to 14 for the control of the efficacy of a therapeutic treatment.

18. Use of the methods of claims 1 to 14 for the design of individualized peptide vaccines for the treatment of diseases.

## Patentansprüche

1. Verfahren zur Isolierung von antigenen Peptiden in femtomolaren Mengen, wobei das Verfahren aus
(a) dem Bereitstellen von Komplexen von MHC-II-Molekülen mit antigenen Peptiden, isoliert aus Zellen, Gewebeproben oder Körperflüssigkeit eines Säugerorganismus, in einer Menge von 0,2 bis 3 µg und
(b) dem Sequestrieren der Komplexe durch Immunpräzipitation, wobei die Komplexe durch MHC-II-spezifische Antikörper an Kügelchen gekoppelt sind;
(c) dem Waschen der gebundenen Komplexe von MHC-II-Molekülen mit antigenen Peptiden mit Wasser oder salzarmem Puffer, wobei der salzarme Puffer ein Tris-, Phosphat- oder Acetatpuffer in einem Konzentrationsbereieh von 0,5 bis 10 mM ist, und
(d) dem Eluieren der assoziierten antigenen Peptide aus den MHC-II-Molekülen besteht.

2. Verfahren nach Anspruch 1, wobei die aus einem Säugerorganismus isolierten Zellen dendritische Zellen sind.

3. Verfahren nach den Ansprüchen 1 oder 2, wobei die sequestrierten Komplexe von MHC-II-Molekülen mit antigenen Peptiden vor der Elution der Peptide in einem Ultrafiltrationsrohr mit Wasser gewaschen werden.

4. Verfahren nach den Ansprüchen 1 bis 3, wobei die antigenen Peptide aus den MHC-II-Molekülen unter Verwendung einer verdünnten Säure eluiert werden.

5. Verfahren nach einem der Ansprüche 1 bis 3, wobei die antigenen Peptide aus den MHC-11-molekülen bei 37°C mit verdünnter Trifluoressigsäure eluiert werden.

6. Verfahren nach den Ansprüche 1 bis 5, wobei die isolierten antigenen Peptide fraktioniert, sequenziert und identifiziert werden.

7. Verfahren nach Anspruch 6, wobei die isolierten antigenen Peptide durch Verfahren, umfassend Flüssigchromatographie und Massenspektrometrie, fraktioniert, sequenziert und identifiziert werden.

8. Verfahren nach den Ansprüchen 1 bis 7, wobei die antigenen Peptide natürlich prozessierte antigene Peptide oder nicht natürlich prozessierte antigene Peptide sind, die dem Organismus verabreicht werden,

9. Verfahren nach den Ansprüchen 1 bis 8, wobei die Zellen Zellen sind, die MHC-II-Moleküle exprimieren.

10. Verfahren nach den Ansprüchen 1 bis 9, wobei der Säugerorganismus ein menschlicher Organismus ist.

11. Verfahren nach einem der Ansprüche 2 bis 10,
wobei die Zellen von Schritt a) MHC-II-exprimierende Zellen in einer Anzahl, die 0,2 bis 3 µg MHC-II-Moleküle liefert, sind, und wobei die MHC-II-exprimierenden Zellen vor der Isolierung der MHC-II-Molekül-Antigenpeptid-Komplexe mit einer Quelle für ein potentielles Antigen kontaktiert werden.

12. Verfahren nach Anspruch 11, wobei die MHC-II-exprimierenden Zellen dendritische Zellen sind, und wobei die dendritschen Zellen einer potentiellen Antigenquelle als unreife dendritische Zellen zu dem Zeitpunkt, wo die Reifung zu dendritischen Zellen induziert wird, ausgesetzt werden.

13. Verfahren nach den Ansprüche 11 bis 12, wobei die Quelle für das potentielle Antigen zu der Gruppe, umfassend Tumorzellen, Tumorzelllinien, Pathogene, virale, bakterielle und parasitische Antigene, Autoantigene, Körperflüssigkeiten, z. B. Serum, Synovia, Aszites, gehört,

14. Verfahren nach den Ansprüchen 11 bis 13, wobei die isolierten antigenen Peptide fraktioniert, sequenziert und identifiziert werden und die antigenen Peptide, abgeleitet von der Quelle für das potentielle Antigen, durch einen Vergleich der Peptide, identifiziert aus Zellen, die mit einer Quelle für ein potentielles Antigen kontaktiert worden sind, mit denen, die aus Zellen identifiziert worden sind, die nicht mit einer Quelle für ein potentielles Antigen kontaktiert worden sind, identifiziert werden.

15. Verwendung der Verfahren nach den Ansprüchen 11 bis 14 zur Qualitätskontrolle von Impfstoffen.

16. Verwendung der Verfahren nach den Ansprüchen 1 bis 14 zur Immunüberwachung von Krankheiten.

17. Verwendung der Verfahren nach den Ansprüchen 1 bis 14 zur Kontrolle der Wirksamkeit einer therapeutischen Behandlung.

18. Verwendung der Verfahren nach den Ansprüchen 1 bis 14 zur Konstruktion individualisierter Peptidimpfstoffe zur Behandlung von Krankheiten.

## Revendications

1. Procédé d'isolement de peptides antigéniques en quantités femtomolaires, ce procédé consistant en
(a) la fourniture de complexes de molécules du CMH II avec des peptides antigéniques isolés à partir de cellules, d'échantillons de tissus ou d'un liquide corporel d'un organisme mammifère en une quantité de 0,2 à 3 µg et
(b) la séquestration des complexes par immunoprécipitation, les complexes étant couplés à des billes par des anticorps spécifiques du CMH II,
(c) le lavage des complexes liés de molécules du CMH II avec des peptides antigéniques avec de l'eau ou un tampon à faible teneur en sels, le tampon à faible teneur en sels étant un tampon Tris, phosphate ou acétate dans une gamme de concentration de 0,5 à 10 mM, et
(d) l'élution des peptides antigéniques associés à partir des molécules du CMH II.

2. Procédé selon la revendication 1, dans lequel les cellules isolées à partir d'un organisme mammifère sont des cellules dendritiques.

3. Procédé selon les revendications 1 ou 2, dans lequel on lave les complexes séquestrés de molécules du CMH II avec des peptides antigéniques avec de l'eau dans un tube d'ultrafiltration avant d'éluer les peptides,

4. Procédé selon les revendications 1 à 3, dans lequel on élue les peptides antigéniques à partir des molécules du CMH II en utilisant un acide dilué.

5. Procédé selon l'une quelconque des revendications 1 à 3, dans lequel on élue les peptides antigéniques à partir des molécules du CMH II à 37°C avec de l'acide trifluoroacétique dilué.

6. Procédé selon les revendications 1 à 5, dans lequel on fractionne, on séquence et on identifie les peptides antigéniques isolés.

7. Procédé selon la revendication 6, dans lequel on fractionne, on séquence et on identifie les peptides antigéniques isolés par des procédés comprenant la chromatographie liquide et la spectrométrie de masse.

8. Procédé selon les revendications 1 à 7, dans lequel les peptides antigéniques sont des peptides antigéniques générés de façon naturelle ou des peptides antigéniques générés de façon non naturelle administrés à l'organisme.

9. Procédé selon les revendications 1 à 8, dans lequel les cellules sont des cellules exprimant les molécules du CMH II.

10. Procédé selon les revendications 1 à 9, dans lequel l'organisme mammifère est un organisme humain.

11. Procédé selon l'une quelconque des revendications 2 à 11, dans lequel les cellules de l'étape a) sont des cellules exprimant le CMH II en un nombre donnant 0,2 à 3 µg de molécules du CMH II et dans lequel on met les cellules exprimant le CMH II en contact avec une source d'antigène potentiel avant d'isoler les complexes molécule du CMH II - peptide antigénique.

12. Procédé selon la revendication 11, dans lequel les cellules exprimant le CMH II sont des cellules dendritiques et dans lequel on expose les cellules dendritiques à une source potentielle d'antigène sous forme de cellules dendritiques immatures en même temps que l'on induit leur maturation en cellules dendritiques.

13. Procédé selon les revendications 11 à 12, dans lequel la source d'antigène potentiel appartient au groupe comprenant des cellules tumorales, des lignées cellulaires tumorales, des pathogènes, des antigènes viraux, bactériens et parasitaires, des auto antigènes, des liquides corporels, par exemple le sérum, le liquide synovial, les ascites.

14. Procédé selon les revendications 11 à 13, dans lequel on fractionne, on séquence et on identifie les peptides antigéniques isolés, et on identifie les peptides antigéniques provenant de la source d'antigène potentiel en comparant les peptides identifiés à partir des cellules qui ont été mises en contact avec une source d'antigène potentiel avec ceux qui ont été identifiés à partir de cellules qui n'ont pas été mises en contact avec une source d'antigène potentiel.

15. Utilisation des procédés des revendications 11 à 14 pour le contrôle de la qualité des vaccins.

16. Utilisation des procédés des revendications 1 à 14 pour le suivi immunologique de maladies.

17. Utilisation des procédés des revendications 1 à 14 pour le contrôle de l'efficacité d'un traitement thérapeutique.

18. Utilisation des procédés des revendications 1 à 14 pour la conception de vaccins peptidiques individualisés destinés au traitement de maladies.
